# FASCICULE DE BREVET EUROPEEN

(11) **EP 1 266 647 B1**
(45) Date de publication et mention de la délivrance du brevet: **16.05.2007**
(21) Numéro de dépôt: 02291465.9
(22) Date de dépôt: 12.06.2002
(51) Int. Cl.: A61K 8/02, A61K 8/19, A61K 8/89, A61K 8/898, A61K 8/899, A61Q 1/02

(54) **Utilisation d'une phase grasse à base d'huile siliconée structurée sous forme rigide pour limiter la migration d'une composition de maquillage**
Verwendung einer stukturierte Fettphase basierend auf einem verfestigten Siliconöl zum begrenzen der Migration einer Schminkzusammensetzung
Use of an oily phase based on a silicon oil structured in rigid form to limit the migration of a make-up composition

(30) Priorité: 14.06.2001 FR 0107776
(43) Date de publication de la demande: 18.12.2002
(60) Demande divisionnaire: 06120057.2
(73) Titulaire: L'ORÉAL, 75008 Paris (FR)
(72) Inventeur: Tournilhac, Florence, 75011 Paris (FR)
(74) Mandataire: Audier, Philippe André

(56) Documents cités:
- EP-A- 0 923 928
- EP-A- 1 048 686
- EP-A- 1 068 856
- WO-A-97/36573
- WO-A-99/06473
- WO-A-99/47111
- FR-A- 2 765 800
- US-A- 5 412 004
- US-A- 5 919 441
- US-A- 5 981 680
- LOCHHEAD R.Y.; GRUBER J.V.: 'APPENDIX: ENCYCLOPEDIA OF POLYMERS AND THICKENERS FOR COSMETICS' COSMETIC SCIENCE AND TECHNOLOGY SERIES vol. 22, 1999, DEKKER, NEW YORK, NY, US, pages 571 - 655, XP001083641

## Description

### Domaine technique

La présente invention se rapporte à une composition de soin et/ou de traitement et/ou de maquillage de la peau, y compris du cuir chevelu, et/ou des lèvres des êtres humains, contenant une phase grasse liquide comprenant au moins une huile siliconée, gélifiée par un polymère particulier, se présentant notamment sous forme d'un produit coulé de maquillage et en particulier d'un stick de maquillage comme les rouges à lèvres, dont l'application conduit à un dépôt brillant et non-migrant.

Elle concerne plus particulièrement des compositions cosmétiques et dermatologiques telles que des produits de maquillage, présentant des propriétés de tenue, de non-transfert et de stabilité.

### Etat de la technique antérieure

Dans les produits cosmétiques ou dermatologiques, il est courant de trouver une phase grasse liquide structurée, à savoir gélifiée et/ou rigidifiée ; ceci est notamment le cas dans les compositions solides comme les déodorants, les baumes et les rouges à lèvres, les fards à paupière, les produits anti-cerne et les fonds de teint coulés. Cette structuration est obtenue à l'aide de cires ou de charges. Malheureusement, ces cires et charges ont tendance à matifier la composition, ce qui n'est pas toujours souhaitable en particulier pour un rouge à lèvres.

Par phase grasse liquide, au sens de la demande, on entend une phase grasse liquide à température ambiante (25°C) et pression atmosphérique (760 mm de Hg), composée d'un ou plusieurs corps gras liquides à température ambiante, appelés aussi huiles, compatibles entre eux et contenant une huile siliconée.

Par phase grasse liquide structurée, au sens de la demande, on entend que cette phase structurée ne s'écoule pas entre les doigts et est au moins épaissie.

La structuration de la phase grasse liquide permet en particulier de limiter son exsudation des compositions solides et, en plus, de limiter, après dépôt sur la peau ou les lèvres, la migration de cette phase dans les rides et ridules, ce qui est particulièrement recherché pour un rouge à lèvres ou un fard à paupières. En effet, une migration importante de la phase grasse liquide, chargée de matières colorantes, conduit à un effet inesthétique autour des lèvres ou des yeux, accentuant particulièrement les rides et les ridules. Cette migration est souvent citée par les femmes comme un défaut majeur des rouges à lèvres classiques, mais aussi des fards à paupières. Par migration, on entend un débordement de la composition déposée sur les lèvres ou la peau en dehors de son tracé initial.

La brillance est liée pour l'essentiel à la nature de la phase grasse liquide. Ainsi, il est possible de diminuer le taux de cires et de charges de la composition pour augmenter la brillance d'un rouge à lèvres, mais alors, la migration de la phase grasse liquide augmente. Autrement dit, les taux de cires et de charges nécessaires à la réalisation d'un stick de dureté convenable sont un frein à la brillance du dépôt.

Le document EP-A-1 068 856 [1] décrit des compositions cosmétiques solides, sans cire, comportant une phase grasse liquide structurée par un polymère, dans lesquelles la phase grasse est principalement une huile non siliconée.

L'utilisation de phases grasses à base d'huiles siliconées permet à ce jour d'obtenir des compositions cosmétiques ayant une longue tenue lorsque les huiles sont peu ou pas volatiles, à savoir une bonne tenue dans le temps, notamment de la couleur (non virage, non palissement), et des compositions non-transfert lorsque les huiles siliconées sont volatiles, à savoir ne se déposant pas sur un support tel qu'un verre, une tasse, un tissu ou une cigarette, placé au contact du film de maquillage.

Actuellement, l'utilisation d'huiles siliconées en cosmétique est limitée par le peu de molécules pouvant gélifier ces milieux et ainsi conduire à des compositions se présentant sous forme solide comme les bâtons de rouge à lèvres ou les fonds de teint coulés par exemple. La mise en oeuvre de compositions cosmétiques dont la phase grasse est majoritairement siliconée conduit dans la plupart des cas à des problèmes de compatibilité avec les ingrédients classiquement utilisés en cosmétique.

Dans les documents US-A-5 874 069 [2], US-A-5 919 441 [3], US-A-6 051 216 [4], WO-A-02/17870 [8] et WO-A-02/17881 [9], on a réalisé des compositions cosmétiques telles que des stick ou des gels de déodorant, comprenant une phase huileuse siliconée gélifiée par une cire à base de polysiloxane et polyamide, ou par un polymère contenant des groupes siloxane et des groupes capables d'interactions hydrogène.

Pour cette utilisation comme déodorant, les problèmes de migration de la phase huileuse dans les rides et ridules, ainsi que de tenue et de non-transfert de la composition ne se posent pas comme dans le cas des produits cosmétiques de maquillage décrits ci-dessus.

### Exposé de l'invention

L'invention a justement pour objet l'utilisation d'une phase grasse liquide comprenant au moins une huile siliconée structurée essentiellement par une quantité suffisante d'au moins un polymère particulier, dans une composition de soin et/ou de maquillage et/ou de traitement de la peau et/ou des lèvres, permettant de remédier à ces inconvénients. Plus précisément, l'utilisation selon l'invention est telle pue définie dans la revendication 1.

De façon surprenante, le demandeur a trouvé que l'utilisation de polymères particuliers permettait de structurer, en l'absence ou en présence de faibles quantités de cire, les phases grasses liquides à base d'huile siliconée, comprenant une huile volatile ayant un point éclair approprié, sous forme de produit de maquillage ou de soin dont l'application conduisait à un film brillant ou mat et non migrant, et de renforcer les propriétés de tenue et/ou de non-transfert de ces produits.

L'invention s'applique non seulement aux produits de maquillage des lèvres comme les rouges à lèvres, les crayons à lèvres, les brillants à lèvres, mais aussi aux produits de soin et/ou de traitement de la peau, y compris du cuir chevelu, et des lèvres, comme les produits en stick de protection solaire de la peau, du visage ou des lèvres ou les baumes à lèvres, aux produits de maquillage de la peau, aussi bien du visage que du corps humain, comme les fonds de teints coulés en stick ou en coupelle, les produits anti-cerne et les produits de tatouage éphémère, aux produits d'hygiène et de nettoyage, notamment en stick, et aux produits de maquillage des yeux comme les eye-liners en particulier sous forme de crayon et les mascaras pains, notamment pains pour fibres kératiniques (cils, sourcils, cheveux).

Le point éclair est la température à laquelle un carburant prend feu au contact d'une flamme.

Avantageusement, l'huile volatile présente un point éclair supérieur à 60°C, de préférence supérieur à 80°C et mieux encore supérieur à 93°C.

De préférence également, l'huile volatile présente un point éclair égal ou inférieur à 160°C et mieux encore égal ou inférieur à 135°C.

Par "huile volatile", on entend tout milieu non aqueux susceptible de s'évaporer au contact de la peau en moins d'une heure, à une température ambiante et pression atmosphérique. Le ou les huiles cosmétiques volatiles, liquides à température ambiante, ont notamment une pression de vapeur, mesurée à température ambiante et pression atmosphérique, allant de 10⁻³ à 300 mm de Hg (0,266 Pa à 40 000 Pa), de préférence de 0,02 mm à 300 mm de Hg (2,66 Pa à 40 000 Pa) et mieux allant de 0,1 à 90 mm de Hg (13 Pa à 12 000 Pa).

Selon l'invention, l'huile volatile peut être une huile siliconée ou non siliconée.

Aussi, l'invention peut être mise en oeuvre avec les différentes phases grasses suivantes :
1) une phase grasse constituée d'un mélange d'huiles comprenant au moins une huile siliconée non volatile et au moins une huile siliconée volatile ;
2) une phase grasse constituée d'un mélange d'huiles comprenant au moins une huile siliconée non volatile et au moins une huile volatile non siliconée ;
3) une phase grasse constituée d'un mélange d'huiles comprenant au moins une huile siliconée non volatile, au moins une huile siliconée volatile et au moins une huile non siliconée volatile ;
4) une phase grasse constituée d'un mélange d'huiles comprenant au moins une huile siliconée volatile, une huile non siliconée, non volatile et éventuellement au moins une huile non siliconée volatile ; et
5) une phase grasse constituée uniquement d'huiles siliconées volatiles.

Dans les cas 1), 2) et 3), le mélange peut comprendre également une huile non siliconée non volatile.

Généralement l'huile volatile représente de 3 à 89,4% du poids total de la composition, de préférence de 5 à 75%, mieux encore 10 à 60%, par exemple 20 à 50% du poids total de la composition.

Selon l'invention, l'huile siliconée volatile peut être choisie parmi les huiles siliconées linéaires ou cycliques ayant un point éclair d'au moins 40°C, telles que les polydiméthyl siloxanes linéaires ou cycliques, les polyméthylphényl siloxanes, les diméthicone copolyols, les alkylméthicone copolyols, la cétyldiméthicone, les silicones à groupes alkylglycéryl éthers, les silicones à groupes amines latéraux et le dilauroyltriméthylol propane siloxysilicate.

A titre d'exemple de telles huiles volatiles, on peut citer les composés donnés dans le tableau 1 ci-dessous.

**Tableau 1**

| **Composé** | **point éclair (°C)** | **viscosité (cSt)** |
|---|---|---|
| Octyltriméthicone | 93 | 1,2 |
| Hexyltriméthicone | 79 | 1,2 |
| Décaméthyl cyclopentasiloxane (cyclopentasiloxane ou D5) | 72 | 4,2 |
| Octaméthylcyclo tétrasiloxane (cyclotétradiméthyl siloxane ou D4) | 55 | 2,5 |
| Dodécaméthylcyclo hexasiloxane (D6) | 93 | 7 |
| Décaméthyltétrasiloxane (L4) | 63 | 1,7 |
| KF 96 A de Shin Etsu | 94 | 6 |
| PDMS (polydiméthylsiloxane) DC 200 (1,5 cSt) de Dow Corning | 56 | 1,5 |
| PDMS DC 200 (2 cSt) de Dow Corning | 87 | 2 |
| PDMS DC 200 (5 cSt) de Dow Corning | 134 | 5 cst |
| PDMS DC 200 (3 cSt) de Dow Corning | 102 | 3 cSt |

Selon l'invention, l'huile non siliconée volatile peut être choisie dans le groupe des huiles hydrocarbonées et des esters et éthers volatils tels que les hydrocarbures volatils comme l'isododécane et l'isohexadécane, les isoparaffines en C₈-C₁₆, les néopentanoate d'isohexyle ou d'isodécyle, leurs mélanges.

A titre d'exemple d'huiles non siliconées volatiles utilisables dans l'invention, on peut citer les composés du tableau 2 qui suit.

**Tableau 2**

| **Composé** | **Point éclair (°C)** |
|---|---|
| Isododécane | 43 |
| Isohexadécane | 102 |
| Néopentanoate d'isodécyle | 118 |
| n-butyléther de propylène glycol | 60 |
| 3-éthoxypropionate d'éthyle | 58 |
| Acétate de méthyléther de | 46 |
| propylène glycol* | |
| Isopar L (isoparaffine C₁₁-C₁₃) | 62 |
| Isopar H (isoparaffine C₁₁-C₁₂) | 56 |

Selon l'invention, ces huiles volatiles facilitent, notamment, l'application de la composition sur la peau, les lèvres ou les phanères. Ces huiles peuvent être des huiles hydrocarbonées, des huiles siliconées comportant éventuellement des groupements alkyle ou alkoxy pendants ou en bout de chaîne siliconée ou un mélange de ces huiles.

Comme huile volatile utilisable dans l'invention, on peut citer les huiles de silicone linéaires ou cycliques ayant une viscosité à température ambiante inférieure ou égale à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'ivnention, on peut citer notamment l'octaméthylcyclo-tétrasiloxane, le décaméthylcyclopentasiloxane, le dodécaméthylcyclohexasiloxane, l'heptaméthyloctyltrisiloxane, l'hexaméthyldisiloxane, le décaméthyl tétrasiloxane, le dodécaméthyl pentasiloxane et leurs mélanges. On peut aussi utiliser comme huile volatile, le produit commercial KF 96A de 6 cS de Shin Etsu ayant un point éclair de 94°C.

Comme autre huile volatile utilisable dans l'invention, on peut citer les huiles volatiles hydrocarbonées ayant de 8 à 16 atomes de carbone et leurs mélanges et notamment les alcanes ramifiés en C₈-C₁₆ comme les isoalcanes (appelées aussi isoparaffines) en C₈-C₁₆, l'isododécane, l'isodécane, l'isohexadécane et par exemple les huiles vendues sous les noms commerciaux d'Isopars ou de PermetyLs, les esters ramifiés en C₈-C₁₆ comme le néo pentanoate d'isohexyle et leurs mélanges.

Avantageusement, la composition contient au moins une huile volatile siliconée de point éclair supérieur à 40°C.

La phase grasse liquide contient avantageusement au moins 40% et mieux encore au moins 50% en poids d'huile(s) siliconée(s) ayant avantageusement une viscosité inférieure à 5 000 cSt et mieux inférieure à 3 000 cSt car les polymères siliconés utilisés dans l'invention sont plus solubles dans les huiles siliconées de faible viscosité. Elle peut contenir également d'autres huiles ou mélange d'huiles non siliconées.

Les huiles siliconées utilisables dans l'invention peuvent être en particulier les polydiméthylsiloxanes (PDMS) volatils ou non, linéaires ou cycliques, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, des diphényl diméthicones, les diphényl méthyldiphényl trisiloxanes, et les 2-phényléthyl triméthylsiloxysilicates.

La phase grasse liquide peut aussi contenir d'autres huiles non siliconées, par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel ;
- les huiles ou esters de synthèse de formule R₅COOR₆ dans laquelle R₅ représente le reste d'un acide gras supérieure linéaire ou ramifié comportant de 1 à 40 et mieux de 7 à 19 atomes de carbone et R₆ représente une chaîne hydrocarbonée ramifiée contenant de 1 à 40 et mieux de 3 à 20 atomes de carbone, avec R₅ + R₆ ≥ 10 comme par exemple l'huile de Purcellin (octanoate de cétostéaryle), l'isononanoate d'isononyle, le benzoate d'alcool en C₁₂ à C₁₅, le myristate d'isopropyle, le palmitate d'éthyl-2-hexyle, des octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en C₈ à C₂₆ comme l'alcool oléique ; et
- leurs mélanges.

La phase grasse liquide peut encore contenir des huiles apolaires telles que les hydrocarbures ou fluorocarbures, linéaires ou ramifiés, d'origine synthétique ou minérale, volatils ou non, comme les huiles de paraffine volatiles (telles que les isoparaffines, l'isododécane) ou non volatiles et ses dérivés, la vaseline, les polydécènes, le polyisobutène hydrogéné tel que le parléam, le squalane, et leurs mélanges.

Généralement, la phase grasse liquide représente de 5 à 99% du poids total de la composition et mieux de 20 à 75%.

Selon l'invention, la composition comprend généralement de plus une matière colorante. Cette matière colorante peut être insoluble dans le milieu, par exemple être constituée de pigments ou de nacres, ou être soluble dans les huiles, par exemple être constituée de colorant(s) liposoluble(s).

Selon l'invention, la composition comprend généralement en outre des particules solides choisies parmi les charges, les pigments et leurs mélanges. Généralement la taille moyenne des particules solides est de 10 nm à 50 µm, de préférence de 50 nm à 30 µm, mieux de 100 nm à 10 µm.

Les charges utilisées dans les compositions cosmétiques ont généralement pour but d'absorber la sueur et le sébum ou d'apporter de la matité. Selon l'invention, elles permettent de plus de structurer la phase grasse liquide comportant une huile siliconée et de renforcer les propriétés de tenue et/ou de non-transfert de la composition ainsi que la stabilité thermique.

Dans une composition anhydre en stick comme dans les rouges à lèvres et les produits anti-cernes en bâton, les charges permettent, en outre, de limiter l'exsudation de l'huile en dehors du bâton même à chaud (45-47°C) et/ou de limiter la migration de la phase grasse liquide en dehors de son tracé original, notamment dans les rides et ridules.

Par pigments, on entend toute particule solide insoluble dans la composition servant à donner et/ou modifier une couleur et/ou aspect irisé.

Ces pigments peuvent à la fois assurer la fonction d'absorption de la sueur et du sébum, et la fonction de coloration ou de modification d'aspect de la composition, soit du produit cosmétique de maquillage, de traitement ou d'hygiène corporelle. Dans l'invention, ils participent également à la structuration de la phase grasse liquide.

Ces charges ou pigments peuvent être soit de nature hydrophobe, soit de nature hydrophile. Lorsque ces charges ou pigments sont des particules hydrophiles, on facilite leur dispersion dans la composition, soit en les enrobant dans un film de composé hydrophobe, soit en ajoutant une silicone amphiphile à la composition, soit en ajoutant un dispersant ou encore en effectuant un greffage d'un composé hydrophobe.

Les pigments ou charges hydrophobes peuvent être constitués par des particules plus ou moins sphériques de polymères ou copolymères hydrophobes réticulés. A titre d'exemple de polymères et copolymères hydrophobes réticulés, on peut citer :
1°) les polymères fluorés tels que les poudres de polytétrafluoroéthylène et les poudres de copolymère de tétrafluoroéthylène et d'oléfine, par exemple d'éthylène ou de propylène ;
2°) les élastomères silicones, par exemples des poudres de polyméthylsilsesquioxane ;
3°) les polyoléfines telles que le polyéthylène ;
4°) les polyméthacrylates d'alkyle, par exemple le polyméthacrylate de méthyle ;
5°) les polyamides ;
6°) les polystyrènes et dérivés ;
7°) les polyesters ;
8°) les polyacryliques ; et
9°) les polyuréthanes, par exemple les poudres d'hexaméthylène diisocyanate (HDI)/triméthylol hexalactone.

Au lieu de particules sphériques, on peut bien entendu utiliser des fibres de nature hydrophobe, en particulier des fibres des polymères et copolymères cités précédemment ainsi que des charges sous forme de plaquettes.

Les particules solides peuvent être aussi constituées par des pigments et/ou des nacres permettant d'obtenir un maquillage couvrant, c'est-à-dire ne laissant pas voir la peau, les lèvres ou les phanères. Ces particules permettent en outre de réduire le toucher collant des compositions.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0 à 40%, de préférence de 1 à 35%, et mieux de 2 à 25% du poids total de la composition.

Les pigments nacrés (ou nacres) peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20% du poids total de la composition et mieux de 0,1 à 15%.

Lorsque les pigments ou les charges sont hydrophiles, on les enrobe dans un film de composé hydrophobe pour les introduire dans la phase grasse liquide de la composition de l'invention, ou on leur fait subir un traitement hydrophobe.

L'enrobage peut consister en un traitement de surface des particules avant leur introduction dans la phase grasse, par exemple lors de leur fabrication, ou in situ.

L'enrobage ou traitement de surface peut être un enrobage fluoré tel qu'un mono- ou diester perfluoroalkyle d'acide phosphorique, (acide ou sel), un perfluoropolyéther, un perfluoroacide carboxylique ou sulfonique, ou un sel de perfluoroalkyl phosphate de diéthanolamine.

L'enrobage peut être un enrobage ou un greffage à base de silicone fluorée, par exemple un greffage par un silane à groupe perfluoroalkyle.

Le traitement de surface peut aussi être effectué au moyen de dérivés siliconés, par exemple un greffage par des silicones réactives possédant initialement des groupes hydrogénosilanes, un greffage par un diorganosilane tel que le diméthylchlorosilane ou par un alkylalcoxysilane, un greffage par un silane à groupe glycydoxypropyle, un enrobage par une silicone polyglycérolée, ou un enrobage par un copolymère acrylique greffé silicone ou silicone-g-polyacrylique.

On peut encore utiliser un enrobage par des N-acylaminoacides, par exemple la N-lauroyllysine, des enrobages par des acides gras ou sels d'acide gras du type acide stéarique, des enrobages par des lécithines et des enrobages par des huiles esters.

En général, la composition contient de 0,1 à 90% de particules solides, par rapport au poids total de la composition, de préférence de 1 à 70%, mieux de 2 à 50%, et par exemple de 5 à 25%.

On peut aussi faciliter la dispersion des particules hydrophiles au moyen d'au moins une silicone amphiphile qui joue le rôle de tensioactif entre les particules hydrophiles et la phase siliconée hydrophobe.

Ces silicones amphiphiles comportent une partie silicone qui est compatible avec le milieu très siliconé des compositions de l'invention, et une partie hydrophile qui peut être, par exemple, le reste d'un composé choisi parmi les alcools et les polyols, ayant de 1 à 12 groupements hydroxyle, les polyoxyalkylènes comportant au moins deux motifs oxyalkylénés et ayant de 0 à 20 motifs oxypropylénés et/ou de 0 à 20 motifs oxyéthylénés. Cette partie hydrophile a donc une affinité pour les particules hydrophiles et favorise leur dispersion dans le milieu siliconé.

La silicone amphiphile peut être une huile sans activité gélifiante. De telles huiles peuvent être constituées par :
- des diméthicone copolyols, comportant éventuellement des groupes phényle,
- des alkylméthicone copolyols,
- des silicones polyglycérolées, c'est-à-dire des silicones à groupes alkylglycéryl éthers,
- des silicones à groupes latéraux perfluorés et à groupes latéraux glycérolés,
- des silicones à groupes latéraux polyoxyéthylénés/polyoxypropylénés et à groupes latéraux perfluorés,
- des copolymères à bloc silicone et à bloc hydrophile autre que polyéther, par exemple polyoxazoline ou polyéthylèneimine,
- des copolymères greffés du type polysaccharides greffés silicone,
- des copolymères à bloc silicone à bloc poly(oxyde d'éthylène/oxyde de propylène).

La silicone amphiphile utilisée dans l'invention peut être aussi une résine silicone amphiphile au moins partiellement réticulée.

A titre d'exemple de telles résines, on peut citer :
- les résines silicones réticulées à groupes alkylpolyéther, tels que polyoxyde d'éthylène (POE) et polyoxyde d'éthylène/polyoxyde de propylène (POE/POP), décrites dans US-A-5 412 004 [6], et
- les résines silicones réticulées partiellement par des α,ω-diènes, possédant à la fois des chaînes latérales POE/POP hydrophiles et des chaînes latérales alkyle hydrophobes telles que celles décrites dans EP-A-1 048 686 [7]. Les chaînes latérales hydrophiles sont obtenues par réaction avec un POE/POP à une seule extrémité vinylique, et les chaînes latérales alkyle sont formées par réaction avec une α-oléfine à chaîne grasse.

La quantité totale de silicone amphiphile peut aller de 0,1 à 20% du poids total de la composition et mieux de 0,1 à 10%.

Dans la résine silicone amphiphile, la partie silicone est avantageusement formée de polydiméthylsiloxane.

Dans la composition de l'invention, le polymère jouant le rôle d'agent gélifiant, représente généralement de 0,5 à 80% de préférence de 2% à 60% et mieux encore de 5 à 40% du poids total de la composition.

Par ailleurs, le rapport massique polymère gélifiant/huile(s) siliconée(s) est de préférence de 0,1 à 50%.

Les polymères utilisés comme agents gélifiants dans la composition de l'invention sont des polymères du type polyorganosiloxane tels que ceux décrits dans les documents US-A-5 874 069 [2], US-A-5, 919, 441 [3], US-A-6, 051, 216 [4] et US-A-5, 981, 680 [5].

Selon l'invention, les polymères utilisés comme agent gélifiant peuvent appartenir aux deux familles suivantes :
1) des polyorganosiloxanes comportant au moins deux groupes capables d'établir des interactions hydrogène, ces deux groupes étant situés dans la chaîne du polymère ; et/ou
2) des polyorganosiloxanes comportant au moins deux groupes capables d'établir des interactions hydrogène, ces deux groupes étant situés sur des greffons ou ramifications.

Les polymères auxquels s'appliquent l'invention sont des solides qui peuvent être solublisés au préalable dans un solvant à interaction hydrogène capable de rompre les interactions hydrogène des polymères comme les alcools inférieurs en C₂ à C₈ et notamment l'éthanol, le n-propanol, l'isopropanol, avant d'être mis en présence des huiles volatiles selon l'invention. Il est aussi possible d'utiliser ces solvants "rupteurs" d'interaction hydrogène comme co-solvant. Ces solvants peuvent ensuite être conservés dans la composition ou bien être éliminés par évaporation sélective, bien connue de l'homme de l'art.

Les polymères comportant deux groupes capables d'établir des interactions hydrogène dans la chaîne du polymère peuvent être des polymères comprenant au moins un motif répondant à la formule : 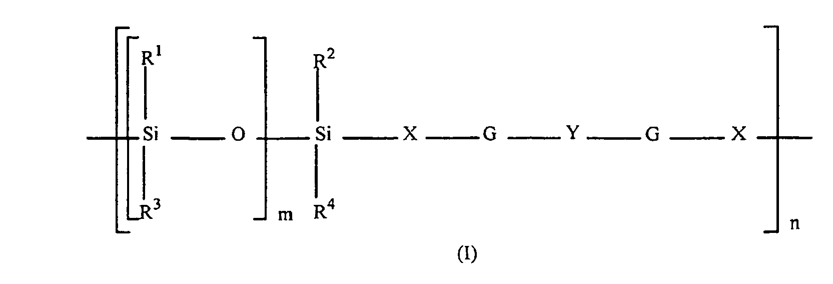 dans laquelle :
1) R¹, R², R³ et R⁴, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote ;
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote ;
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 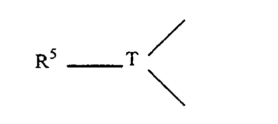 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
   - R⁵ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 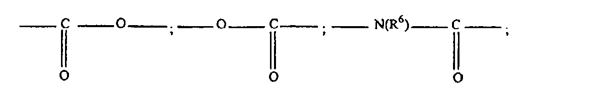 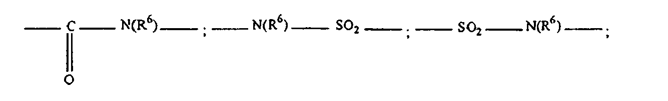 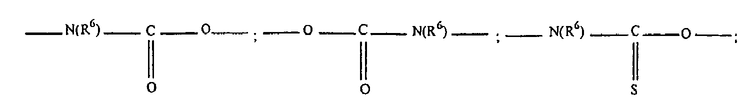 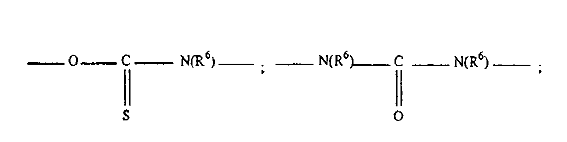 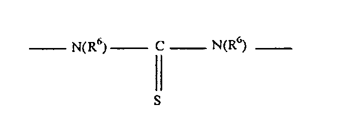 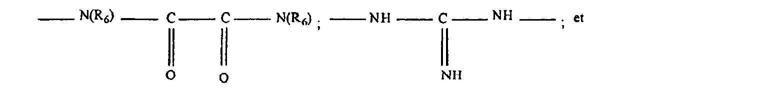 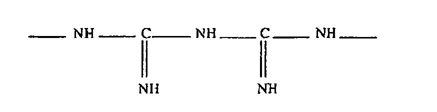 où R⁶ représente un atome d'hydrogène ou un groupe alkyle, linéaire ou ramifié, en C₁ à C₂₀, à condition qu'au moins 50% des R⁶ du polymère représente un atome d'hydrogène et qu'au moins deux des groupes G du polymère soient un autre groupe que : 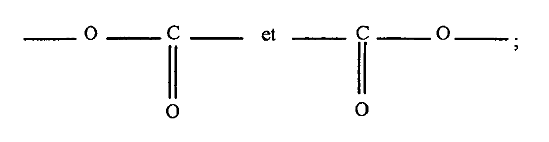
6) n est un nombre entier allant de 2 à 500, de préférence de 2 à 200, et m est un nombre entier allant de 1 à 1000, de préférence de 1 à 700 et mieux encore de 6 à 200.

Selon l'invention, 80% des R¹, R², R³ et R⁴, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆,
g) les chaînes polyorganosiloxane de formule : 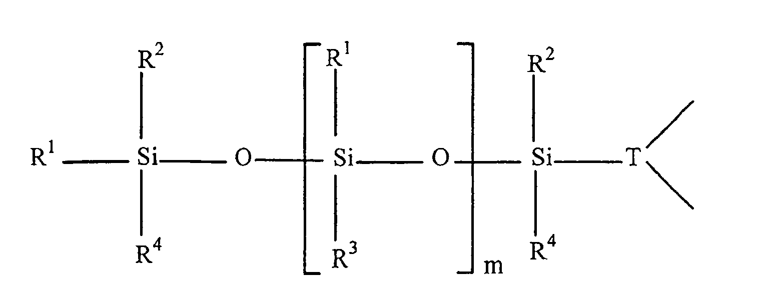 dans laquelle R¹, R², R³, R⁴, T et m sont tels que définis ci-dessus, et
h) les chaînes polyorganosiloxanes de formule :

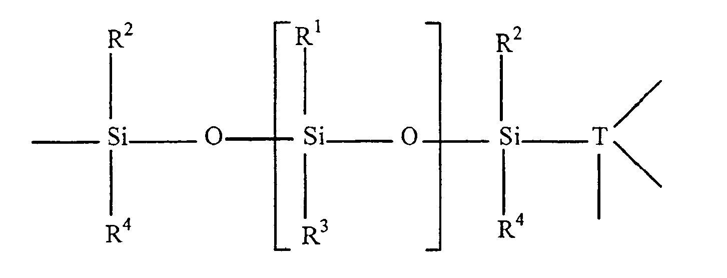

Les polyorganosiloxanes de la seconde famille peuvent être des polymères comprenant au moins un motif répondant à la formule (II) : 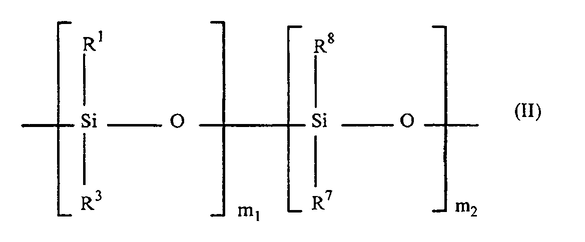 dans laquelle
- R¹ et R³, identiques ou différents, sont tels que définis ci-dessus pour la formule (I),
- R⁷ représente un groupe tel que défini ci-dessus pour R¹ et R³, ou représente le groupe de formule -X-G-R⁹ dans laquelle X et G sont tels que définis ci-dessus pour la formule (I) et R⁹ représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
- R⁸ représente le groupe de formule -X-G-R⁹ dans laquelle X, G et R⁹ sont tels que définis ci-dessus,
- m₁ est un nombre entier allant de 1 à 998, et
- m₂ est un nombre entier allant de 2 à 500.

Selon l'invention, le polymère utilisé comme agent gélifiant, peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des R¹, R², R³, R⁴, X, G, Y, m et n est différent dans l'un des motifs. Le copolymère peut être aussi formé de plusieurs motifs de formule (II), dans lequel l'un au moins des R¹, R³, R⁷, R⁸, m₁ et m₂ est différent dans l'un au moins des motifs.

On peut encore utiliser un copolymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, thiourée et leurs combinaisons. Ces copolymères peuvent être des copolymères blocs, des copolymères séquencés ou des copolymères greffés.

Selon un premier mode de réalisation de l'invention, les groupes capables d'établir des interactions hydrogènes sont des groupes amides de formule -C(O)NH- et -HN-C(O)-.

Dans ce cas, l'agent gélifiant peut être un polymère comprenant au moins un motif de formule (III) ou (IV) : 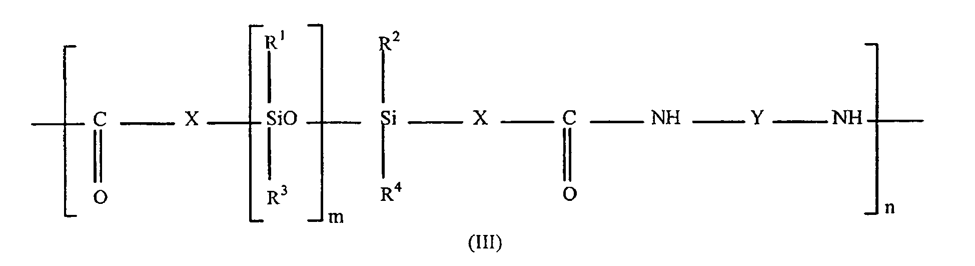 ou 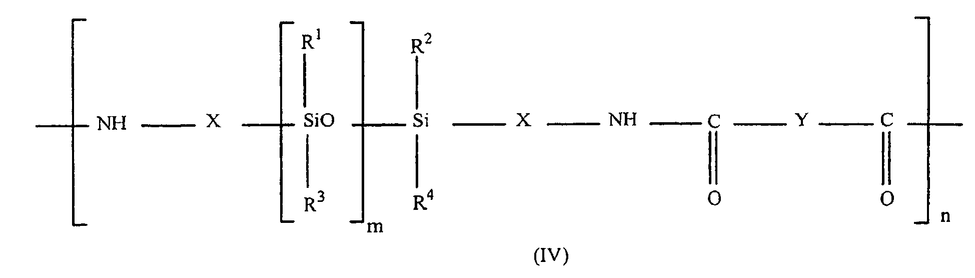 dans lesquelles R¹, R², R³, R⁴, X, Y, m et n sont tels que définis ci-dessus.

Un tel motif peut être obtenu :
- soit par une réaction de condensation entre un silicone à extrémités α, ω-acides carboxyliques et une
ou plusieurs diamines, selon le schéma réactionnel suivant : 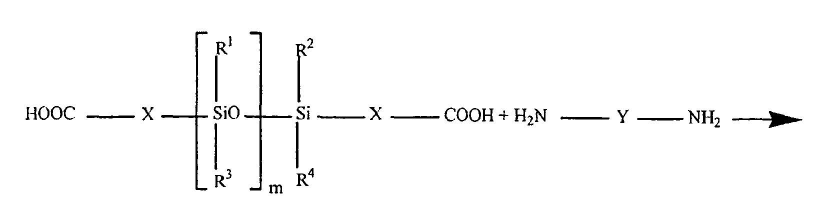 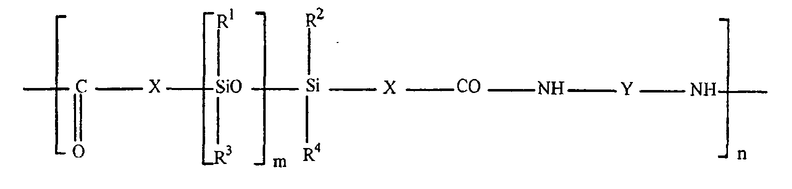
- soit par réaction de deux molécules d'acide carboxylique α-insaturé avec une diamine selon le schéma réactionnel suivant : 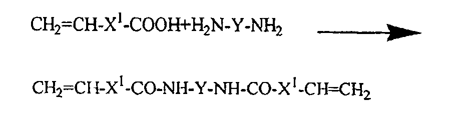
suivie de l'addition d'un siloxane sur les insaturations éthyléniques, selon le schéma suivant :

CH₂=CH-X¹-CO-NH-Y-NH-CO-X¹-CH=CH₂

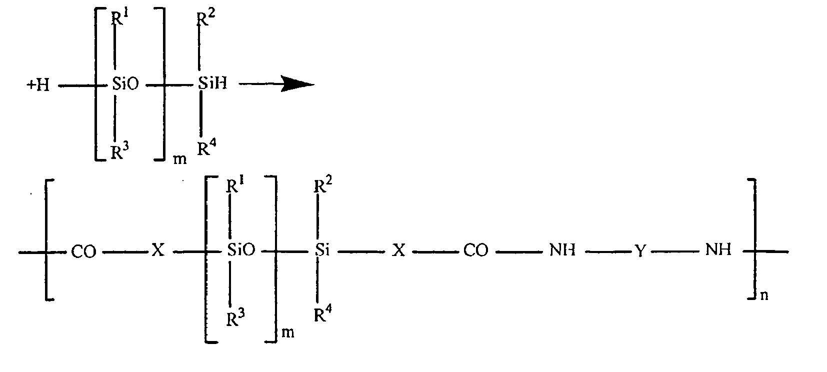

dans lesquels X¹-(CH₂)₂- correspond au X défini ci-dessus et Y, R¹, R², R³, R⁴ et m sont tels que définis ci-dessus ;
- soit par réaction d'un silicone à extrémités α, ω-NH₂ et d'un diacide de formule HOOC-Y-COOH selon le schéma réactionnel suivant :

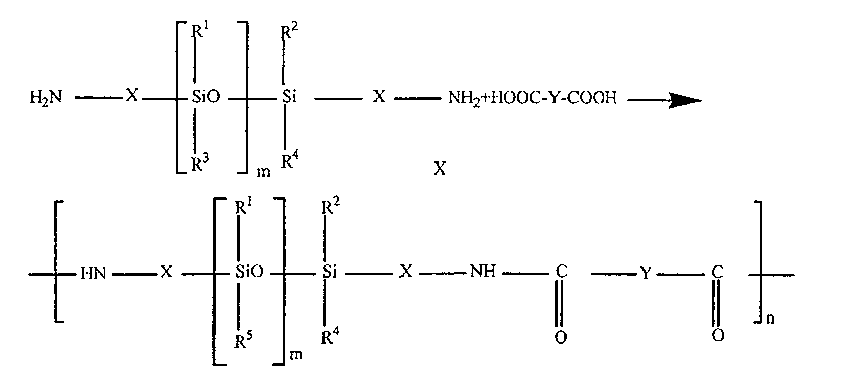

Dans ces polyamides de formule (III) ou (IV), m est de préférence dans la gamme de 1 à 700, de préférence de 15 à 500 et mieux encore de 15 à 45, et n est en particulier dans la gamme de 1 à 500, de préférence de 1 à 100 et mieux encore de 4 à 25,
X est de préférence une chaîne alkylène linéaire ou ramifiée ayant de 1 à 30 atomes de carbone, en particulier 3 à 10 atomes de carbone, et
- Y est de préférence une chaîne alkylène linéaire ou ramifiée ou pouvant comporter des cycles et/ou des insaturations ayant de 1 à 40 atomes de carbone, en particulier de 1 à 20 atomes de carbone, et mieux encore de 2 à 6 atomes de carbone, en particulier de 6 atomes de carbone.

Dans les formules (III) et (IV), le groupe alkylène représentant X ou Y peut éventuellement contenir dans sa partie alkylène au moins l'un des éléments suivants :
1°) 1 à 5 groupes amides, urée ou carbamate,
2°) un groupe cycloalkyle en C₅ ou C₆, et
3°) un groupe phénylène éventuellement substitué par 1 à 3 groupes alkyles identiques ou différents en C₁ à C₃.

Dans les formules (III) et (IV), les groupes alkylènes peuvent aussi être substitués par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

Dans ces formules (III) et (IV), Y peut aussi représenter : 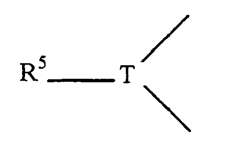 où R⁵ représente une chaîne polyorganosiloxane, et T représente un groupe de formule : 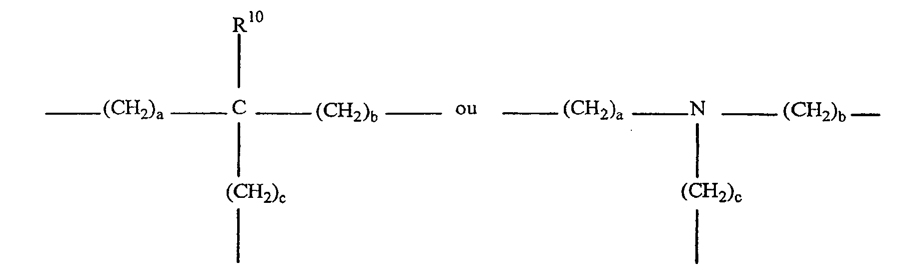 dans lesquelles a, b et c sont indépendamment des nombres entiers allant de 1 à 10, et R¹⁰ est un atome d'hydrogène ou un groupe tel que ceux définis pour R¹, R², R³ et R⁴.

Dans les formules (III) et (IV), R¹, R², R³ et R⁴ représentent de préférence, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (III) ou (IV) identiques ou différents.

Ainsi, le polymère peut être un polyamide contenant plusieurs motifs de formule (III) ou (IV) de longueurs différentes, soit un polyamide répondant à la formule : 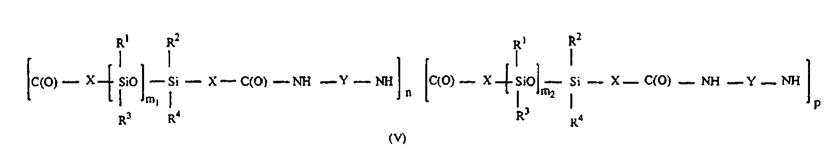 dans laquelle X, Y, n, R¹ à R⁴ ont les significations données ci-dessus, m₁ et m₂ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le copolymère peut répondre à la formule : 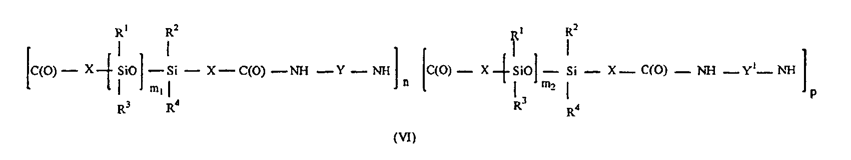 dans laquelle R¹ à R⁴, X, Y, m₁, m₂, n et p ont les significations données ci-dessus et Y¹ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, l'agent gélifiant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le copolymère peut comprendre au moins un motif de formule : 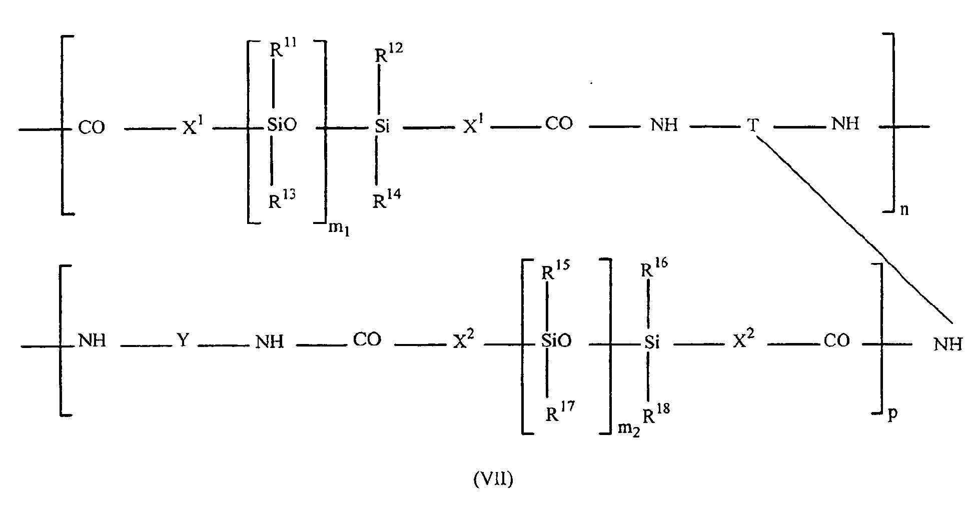 dans laquelle X¹ et X² qui sont identiques ou différents, ont la signification donnée pour X dans la formule (I), n est tel que défini dans la formule (I), Y et T sont tels que définis dans la formule (I), R¹¹ à R¹⁸ sont des groupes choisis dans le même groupe que les R¹ à R⁴, m₁ et m₂ sont des nombres situés dans la gamme allant de 1 à 1 000, et p est un nombre entier allant de 2 à 500.

Dans la formule (VII), on préfère que :
- p soit dans la gamme de 1 à 25, mieux encore de 1 à 7,
- R¹¹ à R¹⁸ soient des groupes méthyle,
- T réponde à l'une des formules suivantes : 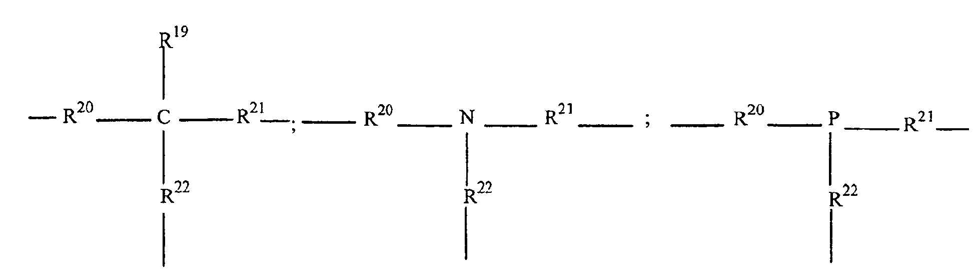 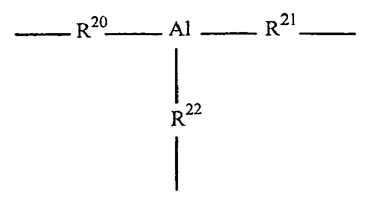
dans lesquelles R¹⁹ est un atome d'hydrogène ou un groupe choisi parmi les groupes définis pour R¹ à R⁴, et R²⁰, R²¹ et R²² sont indépendamment des groupes alkylène, linéaires ou ramifiés, de préférence encore, à la formule : 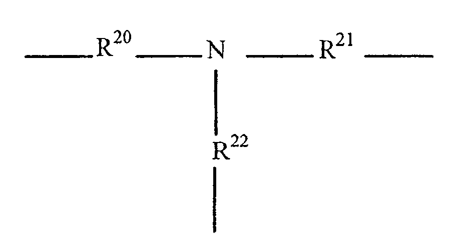 en particulier avec R²⁰, R²¹ et R²² représentant -CH₂-CH₂-,
- m₁ et m₂ soient dans la gamme de 15 à 500, et mieux encore de 15 à 45,
- X¹ et X² représentent (CH₂)₁₀-, et
- Y représente -CH₂-.

Ces polyamides à motif silicone greffé de formule (VII) peuvent être copolymérisés avec des polyamides-silicones de formule (II) pour former des copolymères blocs, des copolymères alternés ou des copolymères aléatoires. Le pourcentage en poids de motifs siliconés greffés (VII) dans le copolymère peut aller de 0,5 à 30% en poids.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon l'invention, les polyamides à base de siloxanes préférés sont :
- les polyamides de formule (III) où m est de 15 à 50 ;
- les mélanges de deux ou plusieurs polyamides dans lesquels au moins un polyamide a une valeur de m dans la gamme de 15 à 50 et au moins un polyamide a une valeur de m dans la gamme de 30 à 50 ;
- des polymères de formule (V) avec m₁ choisi dans la gamme de 15 à 50 et m₂ choisi dans la gamme de 30 à 500 avec la partie correspondant à m₁ représentant 1 à 99% en poids du poids total du polyamide et la partie correspondant à m₂ représentant 1 à 99% en poids du poids total du polyamide ;
- des mélanges de polyamide de formule (III) combinant
   1) 80 à 99% en poids d'un polyamide où n est égal à 2 à 10, en particulier 3 à 6, et
   2) 1 à 20% d'un polyamide où n est dans la gamme de 5 à 500, en particulier de 6 à 100 ;
- des polyamides répondant à la formule (VI) où au moins l'un des Y et Y¹ contient au moins un substituant hydroxyle.
- des polyamides de formule (III) synthétisés avec au moins une partie d'un diacide activé (chlorure, dianhydride ou diester de diacide) au lieu du diacide ;
- des polyamides de formule (III) où X représente
- (CH₂)₃- ou -(CH₂)₁₀ ; et
- des polyamides de formule (III) où les polyamides sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Selon l'invention, les extrémités des chaînes du polymère peuvent être terminées par :
- un groupe ester d'alkyle en C₁ à C₅₀ en introduisant en cours de synthèse un monoalcool en C₁ en C₅₀,
- un groupe amide d'alkyle en C₁ à C₅₀ en prenant comme stoppeur un monoacide si la silicone est α, ω-diaminée, ou une monoamine si la silicone est α, ω-diacide carboxylique.

Selon une variante de réalisation de l'invention, on peut utiliser un copolymère de polyamide silicone et de polyamide hydrocarboné, soit un copolymère comportant des motifs de formule (III) ou (IV) et des motifs polyamide hydrocarboné. Dans ce cas, les motifs polyamide-silicone peuvent être disposés aux extrémités du polyamide hydrocarboné.

Des agents gélifiants à base de polyamide contenant des silicones peuvent être produits par amidation silylique de polyamides à base de dimère d'acide gras. Cette approche implique la réaction de sites acides libres existant sur un polyamide comme sites terminaux, avec des oligosiloxanes-monoamines et/ou des oligosiloxanes-diamines (réaction d'amidation), ou alternativement avec des oligosiloxanes alcools ou des oligosiloxanes diols (réaction d'estérification). La réaction d'estérification nécessite la présence de catalyseurs acides, comme il est connu dans la technique. Il est souhaitable que le polyamide ayant des sites acides libres, utilisés pour la réaction d'amidation ou d'estérification, ait un nombre relativement élevé de terminaisons acides (par exemple des polyamides ayant des indices d'acide élevés, par exemple de 15 à 20).

Pour l'amidation des sites acides libres des polyamides hydrocarbonés, des siloxanes diamines avec 1 à 300, plus particulièrement 2 à 50, et mieux encore 2, 6, 9, 5, 12, 13,5, 23 ou 31 groupes siloxanes, peuvent être utilisés pour la réaction avec des polyamides hydrocarbonés à base de dimères d'acide gras. On préfère des siloxanes diamines ayant 13,5 groupes siloxanes et les meilleurs résultats sont obtenus avec la siloxane-diamine ayant 13,5 groupes siloxane et des polyamides contenant des indices élevés de groupes terminaux acides carboxyliques.

Les réactions peuvent être effectuées dans le xylène pour extraire l'eau produite de la solution par distillation azéotropique, ou à des températures plus élevées (autour de 180 à 200°C) sans solvant. Typiquement, l'efficacité de l'amidation et les taux de réaction diminuent lorsque le siloxane diamine est plus long, c'est-à-dire lorsque le nombre de groupes siloxanes est plus élevé. Des sites amines libres peuvent être bloqués après la réaction d'amidation initiale des diaminosiloxanes en les faisant réagir avec soit un siloxane acide, soit un acide organique tel que l'acide benzoïque.

Pour l'estérification des sites acides libres sur les polyamides, ceci peut être réalisé dans le xylène bouillant avec environ 1% en poids, par rapport au poids total des réactifs, d'acide paratoluènesulfonique comme catalyseur.

Ces réactions effectuées sur les groupes acides carboxyliques terminaux du polyamide conduisent à l'incorporation de motifs silicone seulement aux extrémités de la chaîne de polymère.

On peut aussi préparer un copolymère de polyamide-silicone, en utilisant un polyamide à groupes amines libres, par réaction d'amidation avec un siloxane contenant un groupe acide.

On peut encore préparer un agent gélifiant à base de copolymère entre un polyamide hydrocarboné et un polyamide siliconé, par transamidation d'un polyamide ayant par exemple un constituant éthylène-diamine, par une oligosiloxane-α, ω-diamine, à température élevée (par exemple 200 à 300°C), pour effectuer une transamidation de sorte que le composant éthylène diamine du polyamide d'origine est remplacé par l'oligosiloxane diamine.

Le copolymère de polyamide hydrocarboné et de polyamide-silicone peut encore être un copolymère greffé comportant un squelette de polyamide hydrocarboné avec des groupes oligosiloxane pendants.

Ceci peut être obtenu par exemple :
- par hydrosilylation de liaisons insaturées dans des polyamides à base de dimères d'acide gras ;
- par silylation des groupes amides d'un polyamide ; ou
- par silylation de polyamides insaturés au moyen d'une oxydation, c'est-à-dire en oxydant les groupes insaturés en alcools ou diols, pour former des groupes hydroxyle que l'on fait réagir avec des acides siloxane carboxyliques ou des siloxanes-alcools. On peut aussi époxyder les sites oléfiniques des polyamides insaturés puis faire réagir les groupes époxy avec des siloxanes-amines ou des siloxanes-alcools.

Selon un second mode de réalisation de l'invention, l'agent gélifiant est constitué par un homopolymère ou copolymère comportant des groupes uréthane ou urée.

Comme précédemment, le polymère peut comporter des motifs polyorganosiloxanes contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante : 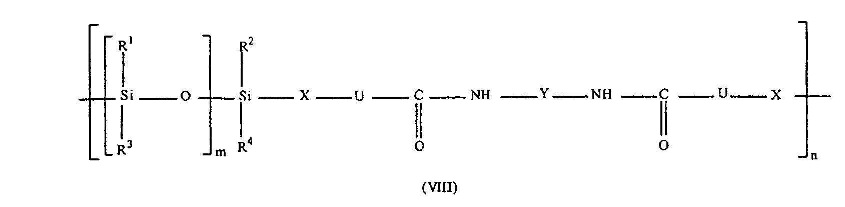 dans laquelle les R¹, R², R³, R⁴, X, Y, m et n ont les significations données ci-dessus pour la formule (I), et U représente -O- ou -NH-, afin que : 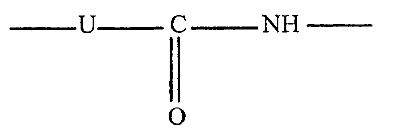 corresponde à un groupe uréthane ou urée.

Dans cette formule (VIII), Y peut être un groupe alkylène, en C₁ à C₄₀, linéaire ou ramifié, substitué éventuellement par un groupe alkyle en C₁ à C₁₅ ou un groupe aryle en C₅ à C₁₀. De préférence, on utilise un groupe - (CH₂)₆-.

Y peut aussi représenter un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane. Généralement, on préfère que Y représente un radical alkylène en C₁ à C₄₀, linéaire ou ramifié, ou un radical cycloalkylène en C₄ à C₁₂.

Y peut aussi représenter une séquence polyuréthane ou polyurée correspondant à la condensation de plusieurs molécules de diisocyanate avec une ou plusieurs molécules de coupleurs du type diol ou diamine. Dans ce cas, Y comprend plusieurs groupes uréthane ou urée dans la chaîne alkylène.

Il peut répondre à la formule : 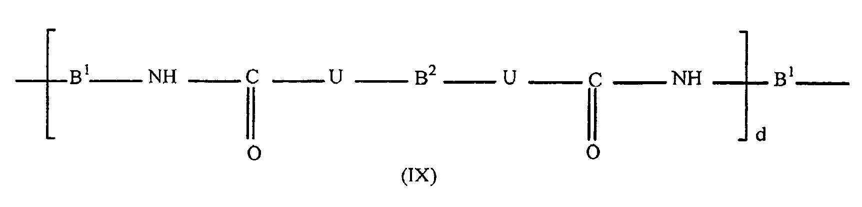 dans laquelle B¹ est un groupe choisi parmi les groupes donnés ci-dessus pour Y, U est -O- ou -NH-, et B² est choisi parmi :
- les groupes alkylène en C₁ à C₄₀, linéaires ou ramifiés, qui peuvent porter éventuellement un groupe ionisable tel qu'un groupe acide carboxylique ou sulfonique, ou un groupe amine tertiaire neutralisable ou quaternisable,
- les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
- les groupes phénylène pouvant éventuellement être porteurs de substituants alkyles en C₁ à C₃, et
- les groupes de formule : 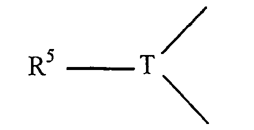
dans laquelle T est un radical trivalent hydrocarboné pouvant contenir un ou plusieurs hétéroatomes tels que l'oxygène, le soufre et l'azote et R⁵ est une chaîne polyorganosiloxane ou une chaîne alkyle en C₁ à C₅₀, linéaire ou ramifiée.

T peut représenter par exemple : 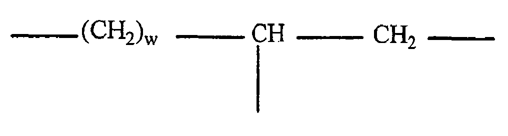 ou 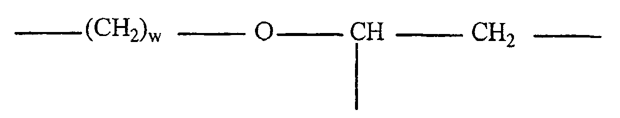 avec w étant un nombre entier allant de 1 à 10 et R⁵ étant une chaîne polyorganosiloxane.

Lorsque Y est un groupe alkylène, en C₁ en C₄₀ linéaire ou ramifié, on préfère les groupes -(CH₂)₂- et -(CH₂)₆-.

Dans la formule donnée ci-dessus pour Y, d peut être un entier allant de 0 à 5, de préférence de 0 à 3, de préférence encore égal à 1 ou 2.

De préférence B² est un groupe alkylène en C₁ à C₄₀, linéaire ou ramifié, en particulier -(CH₂)₂- ou -(CH₂)₆-, ou le groupe : 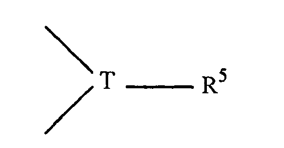 avec R⁵ étant une chaîne polyorganosiloxane.

Comme précédemment, le polymère constituant l'agent gélifiant peut être formé de motifs silicone uréthane et/ou silicone-urée de longueur et/ou de constitution différentes, et se présenter sous la forme de copolymères blocs, séquencés ou aléatoires.

Selon l'invention, le silicone peut aussi comporter les groupes uréthane et/ou urée non plus dans le squelette mais en ramifications latérales.

Dans ce cas, le polymère peut comprendre au moins un motif de formule : 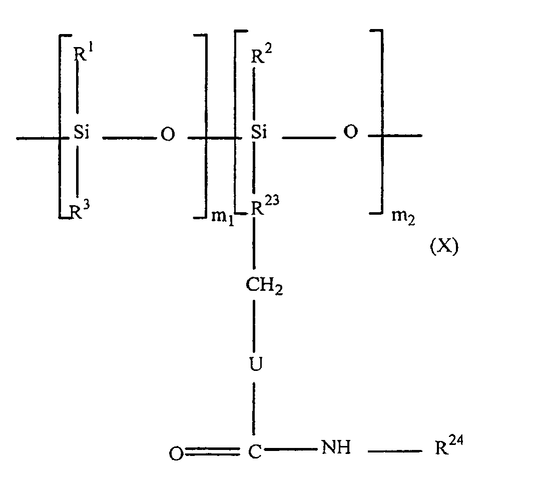 dans laquelle R¹, R², R³, m₁ et m₂ ont les significations données ci-dessus pour la formule (I),
- U représente O ou NH,
- R²³ représente un groupe alkylène en C₁ à C₄₀, comportant éventuellement un ou plusieurs hétéroatomes choisis parmi O et N, ou un groupe phénylène, et
- R²⁴ est choisi parmi les groupes alkyle en C₁ à C₅₀, linéaires, ramifiés ou cycliques, saturés ou insaturés, et les groupes phényle éventuellement substitués par un à trois groupes alkyles en C₁ à C₃.

Les polymères comportant au moins un motif de formule (X) contiennent des unités siloxanes et des groupes urées ou uréthanes, et ils peuvent être utilisés comme agents gélifiants dans les compositions de l'invention.

Les polymères siloxanes peuvent avoir un seul groupe urée ou uréthane par ramification ou peuvent avoir des ramifications à deux groupes urée ou uréthane, ou encore contenir un mélange de ramifications à un groupe urée ou uréthane et de ramifications à deux groupes urée ou uréthane.

Ils peuvent être obtenus à partir de polysiloxanes ramifiés, comportant un ou deux groupes amino par ramification, en faisant réagir ces polysiloxanes avec des monoisocyanates.

A titre d'exemples de polymères de départ de ce type ayant des ramifications amino et diamino, on peut citer les polymères répondant aux formules suivantes :

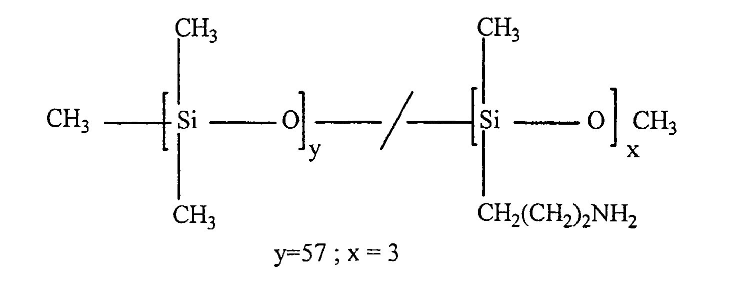

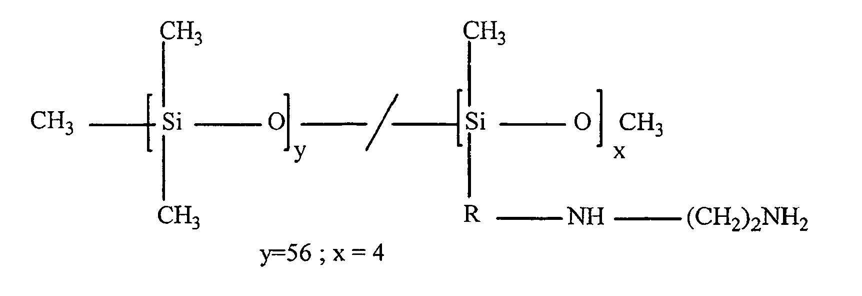

Dans ces formules, le symbole "/" indique que les segments peuvent être de longueurs différentes et dans un ordre aléatoire, et R représente un groupe aliphatique linéaire ayant de préférence 1 à 6 atomes de carbone et mieux encore 1 à 3 atomes de carbone.

De tels polymères à ramification peuvent être formés en faisant réagir un polymère siloxane, ayant au moins trois groupes amino par molécule de polymère, avec un composé ayant un seul groupe monofonctionnel (par exemple un acide, un isocyanate ou isothiocyanate) pour faire réagir ce groupe monofonctionnel avec l'un des groupes amino et former les groupes capables d'établir des interactions hydrogène. Les groupes amino peuvent être sur des chaînes latérales s'étendant de la chaîne principale du polymère siloxane de sorte que les groupes capables d'établir des interactions hydrogène sont formés sur ces chaînes latérales, ou bien les groupes amino peuvent être aux extrémités de la chaîne principale de sorte que les groupes capables d'interaction hydrogène seront des groupes terminaux du polymère.

Comme mode opératoire pour former un polymère contenant des unités siloxanes et des groupes capables d'établir des interactions hydrogène, on peut citer la réaction d'une siloxane diamine et d'un diisocyanate dans un solvant siliconé de façon à fournir directement un gel. La réaction peut être exécutée dans un fluide siliconé, le produit résultant étant dissous dans le fluide siliconé, à température élevée, la température du système étant ensuite diminuée pour former le gel.

Les polymères préférés pour l'incorporation dans les compositions selon la présente invention, sont des copolymères siloxanes-urées qui sont linéaires et qui contiennent des groupes urées comme groupes capables d'établir des interactions hydrogène dans le squelette du polymère.

A titre d'illustration d'un polysiloxane terminé par quatre groupes urées, on peut citer le polymère de formule : 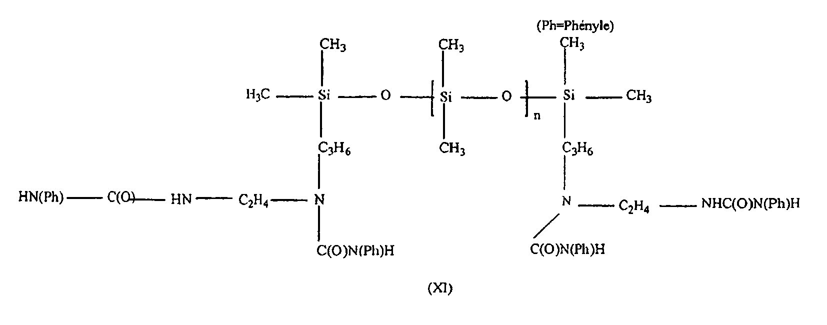 où Ph est un groupe phényle et n est un nombre de 0 à 300, en particulier de 0 à 100, par exemple de 50.

Ce polymère est obtenu par réaction du polysiloxane à groupes amino suivant : 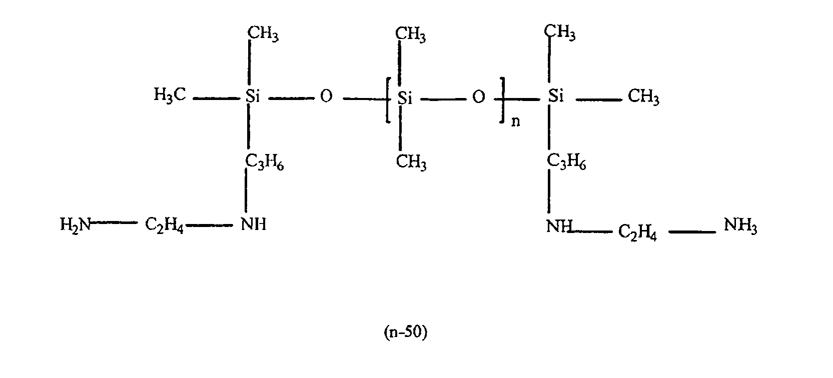 avec l'isocyanate de phényle.

Les polymères de formule (VIII) comportant des groupes urées ou uréthanes dans la chaîne du polymère siliconé peuvent être obtenus par réaction entre un silicone à groupes terminaux α,ω-NH₂ ou -OH, de formule : 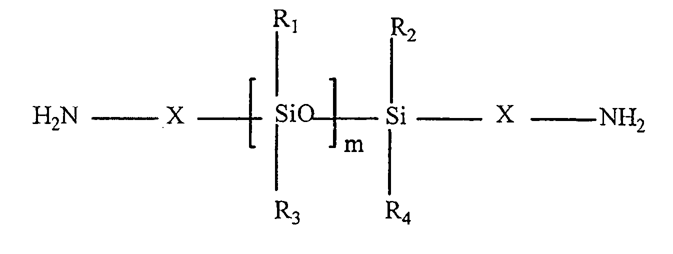 dans laquelle m, R¹, R², R³, R⁴ et X sont tels que définis pour la formule (I), et un diisocyanate OCN-Y-NCO où Y a la signification donnée dans la formule (I) ; et éventuellement un coupleur diol ou diamine de formule H₂N-B²-NH₂ ou HO-B²-OH, où B² est tel que défini dans la formule (IX).

Suivant les proportions stoechiométriques entre les deux réactifs, diisocyanate et coupleur, on pourra avoir pour Y la formule (IX) avec d égal 0 où d égal 1 à 5.

Comme dans le cas des polyamides silicones de formule (II) ou (III), on peut utiliser dans l'invention des polyuréthanes ou des polyurées silicones ayant des motifs de longueur et de structure différentes, en particulier des motifs de longueurs différentes par le nombre d'unités silicones. Dans ce cas, le copolymère peut répondre par exemple à la formule : 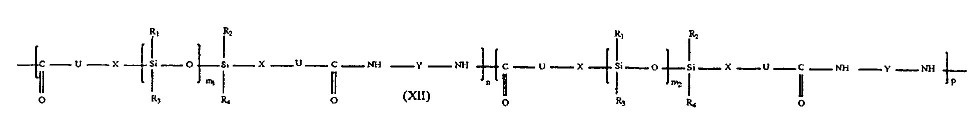 dans laquelle R¹, R², R³, R⁴, X, Y et U sont tels que définis pour la formule (VIII) et m₁, m₂, n et p sont tels que définis pour la formule (V).

On peut obtenir également des polyuréthanes ou polyurées silicones ramifiés en utilisant à la place du diisocyanate OCN-Y-NCO, un triisocyanate de formule :

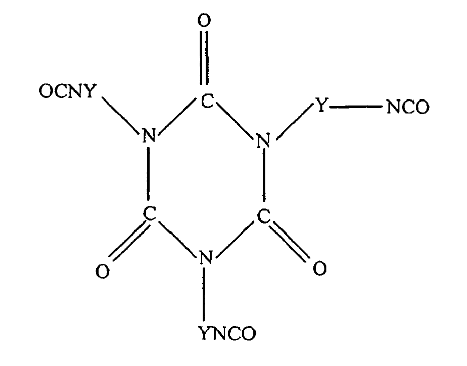

On obtient ainsi une polyuréthane ou polyurée silicone ayant des ramifications comportant une chaîne organosiloxane avec des groupes capables d'établir des interactions hydrogène. Un tel polymère comprend par exemple un motif répondant à la formule : 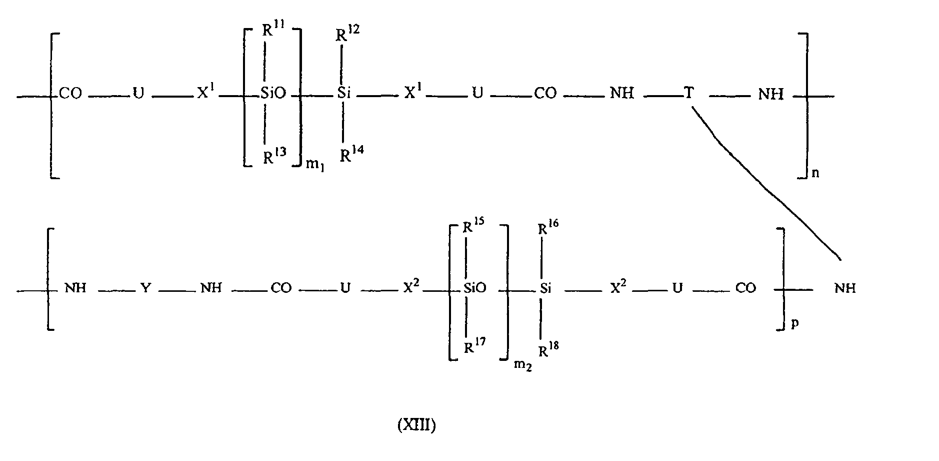 dans laquelle X¹ et X² qui sont identiques ou différents, ont la signification donnée pour X dans la formule (I), n est tel que défini dans la formule (I), Y et T sont tels que définis dans la formule (I), R¹¹ à R¹⁸ sont des groupes choisis dans le même groupe que les R¹ à R⁴, m₁ et m₂ sont des nombres situés dans la gamme allant de 1 à 1 000, et p est un nombre entier allant de 2 à 500.

Comme dans le cas des polyamides, ce copolymère peut comporter aussi des motifs polyuréthanes silicones sans ramification.

Dans ce second mode de réalisation de l'invention, les polyurées et les polyuréthanes à base de siloxanes préférés sont :
- les polymères de formule (VIII) où m est de 15 à 50 ;
- les mélanges de deux ou plusieurs polymères dans lesquels au moins un polymère a une valeur de m dans la gamme de 15 à 50 et au moins un polymère a une valeur de m dans la gamme de 30 à 50 ;
- des polymères de formule (XII) avec m₁ choisi dans la gamme de 15 à 50 et m₂ choisi dans la gamme de 30 à 500 avec la partie correspondant à m₁ représentant 1 à 99% en poids du poids total du polymère et la partie correspondant à m₂ représentant 1 à 99% en poids du poids total du polymère ;
- des mélanges de polymère de formule (VIII) combinant
   1) 80 à 99% en poids d'un polymère où n est égal à 2 à 10, en particulier 3 à 6, et
   2) 1 à 20% d'un polymère où n est dans la gamme de 5 à 500, en particulier de 6 à 100,
- des copolymères comprenant deux motifs de formule (VIII) où au moins l'un des Y contient au moins un substituant hydroxyle ;
- des polymères de formule (VIII) synthétisés avec au moins une partie d'un diacide activé (chlorure, dianhydride ou diester de diacide) au lieu du diacide ;
- des polymères de formule (VIII) où X représente -(CH₂)₃- ou -(CH₂)₁₀- ; et
- des polymères de formule (VIII) où les polymères sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Comme dans le cas des polyamides, on peut utiliser dans l'invention des copolymères de polyuréthane -ou de polyurée- silicone et de polyuréthane ou polyurée hydrocarboné en réalisant la réaction de synthèse du polymère en présence d'une séquence α, ω-difonctionnelle de nature non silicone, par exemple un polyester, un polyéther ou une polyoléfine.

Comme on l'a vu précédemment, les agents gélifiants constitués par des homopolymères ou copolymères de l'invention peuvent avoir des motifs siloxanes dans la chaîne principale du polymère et des groupes capables d'établir des interactions hydrogène, soit dans la chaîne principale du polymère ou aux extrémités de celle-ci, soit sur des chaînes latérales
ou ramifications de la chaîne principale. Ceci peut correspondre aux cinq dispositions suivantes : 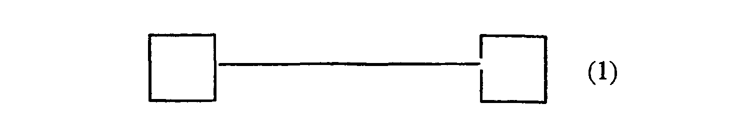 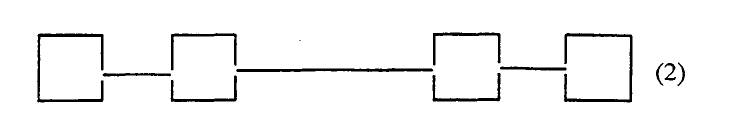 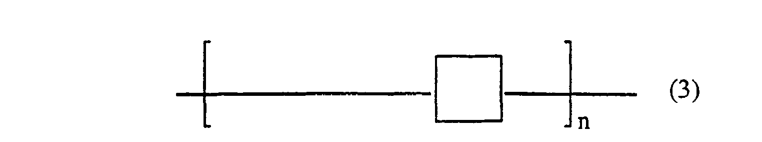 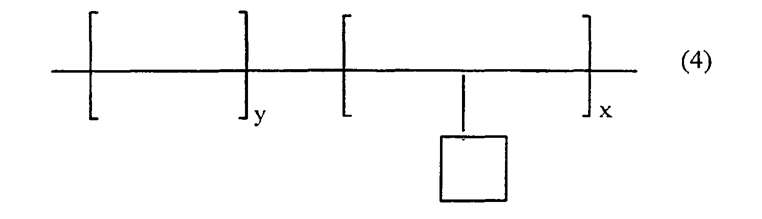 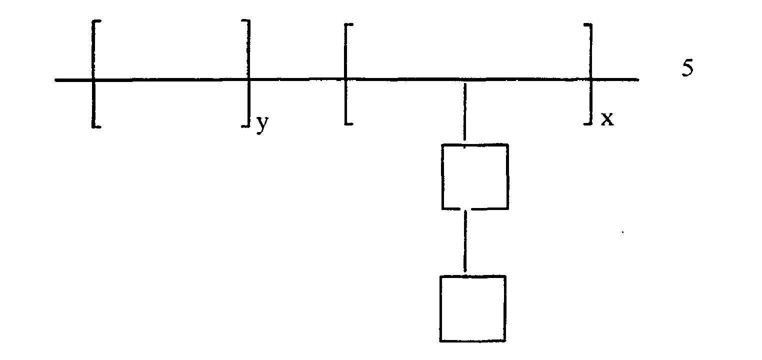 dans lesquelles, la ligne continue est la chaîne principale du polymère siloxane et les carrés représentent les groupes capables d'établir des interactions hydrogène.

Dans le cas (1), les groupes capables d'établir des interactions hydrogène sont disposés aux extrémités de la chaîne principale. Dans le cas (2), deux groupes capables d'établir des interactions hydrogène, sont disposés à chacune des extrémités de la chaîne principale.

Dans le cas (3), les groupes capables d'établir des interactions hydrogène sont disposés à l'intérieur de la chaîne principale dans des motifs répétitifs.

Dans les cas (4) et (5), il s'agit de copolymères dans lesquels les groupes capables d'établir des interactions hydrogène sont disposés sur des ramifications de la chaîne principale d'une première série de motifs qui sont copolymérisés avec des motifs ne comportant pas de groupes capables d'établir des interactions hydrogène. Les valeurs n, x et y sont telles que le polymère présente les propriétés voulues en tant qu'agent gélifiant de phases grasses à base d'huile siliconée.

Selon l'invention, la structuration de la phase grasse liquide contenant au moins une huile siliconée et incluant une huile volatile, siliconée ou non, ayant un point éclair égal ou supérieur à 40°C et supérieur à la température de ramollissement du polymère gélifiant, de préférence supérieur à 60°C et mieux supérieur à 80°C, est obtenue à l'aide d'un ou plusieurs des polymères mentionnés ci-dessus.

A titre d'exemples de polymères utilisables, on peut citer les polyamides siliconés, obtenus conformément aux exemples 1 et 2 du document US-A-5 981 680.

Les polymères et copolymères utilisés comme agents gélifiants dans la composition de l'invention ont avantageusement une température de ramollissement de 40 à 190°C. De préférence, ils présentent une température de ramollissement allant de 50 à 140°C et mieux de 70°C à 120°C. Cette température de ramollissement est plus basse que celle des polymères structurants connus, ce qui facilite la mise en oeuvre des polymères objet de l'invention, permet l'emploi d'huiles volatiles ayant un point éclair supérieur à la température de ramollissement du polymère, et limite les détériorations de la phase grasse liquide.

Grâce au choix d'un polymère gélifiant et d'une huile volatile présentant respectivement une température de ramollissement et un point éclair situés dans les gammes précitées, on évite tout danger d'explosion, lors de l'incorporation du polymère gélifiant dans la composition. En effet, on peut obtenir la fusion du polymère gélifiant à une température inférieure à la température à laquelle l'huile volatile risquerait de prendre feu.

Ainsi, dans l'invention, on choisit le polymère gélifiant et l'huile volatile de façon à respecter les conditions précitées.

Les polymères gélifiants présentent une bonne solubilité dans les huiles siliconées et conduisent à des compositions macroscopiquement homogènes. De préférence, ils ont une masse moléculaire moyenne de 500 à 200 000, par exemple de 1 000 à 100 000 de préférence de 2 000 à 30 000.

Selon l'invention, la composition a de préférence une dureté allant de 20 à 2 000 gf et mieux de 20 à 900 gf, notamment de 20 à 600 gf et par exemple de 150 à 450 gf. Cette dureté peut être mesurée selon une méthode de pénétration d'une sonde dans ladite composition et en particulier à l'aide d'un analyseur de texture (par exemple TA-TXT2ᵢ de chez Rhéo) équipé d'un cylindre en ébonite de 25 mm de haut et 8 mm de diamètre. La mesure de dureté est effectuée à 20°C au centre de cinq échantillons de ladite composition. Le cylindre est introduit dans chaque échantillon de composition à une pré-vitesse de 2 mm/s puis à une vitesse de 0,5 mm/s et enfin à une post-vitesse de 2 mm/s, le déplacement total étant de 1 mm. La valeur relevée de la dureté est celle du pic maximum. L'erreur de mesure est de ± 50 gf.

La dureté peut aussi être mesurée par la méthode dite du fil à couper le beurre, qui consiste à couper un bâton de rouge à lèvres de 8,1 mm de diamètre et à mesurer la dureté à 20°C, au moyen d'un dynamomètre DFGHS 2 de la société Indelco-Chatillon se déplaçant à une vitesse de 100 mm/minute. Elle est exprimée comme la force de cisaillement (exprimée en grammeforce) nécessaire pour couper un stick dans ces conditions. Selon cette méthode la dureté d'une composition en stick selon l'invention va de 30 à 300 gf, de préférence de 30 à 200 gf et par exemple de 30 à 120 gf.

La dureté de la composition selon l'invention est telle que la composition est autoportée et peut se déliter aisément pour former un dépôt satisfaisant sur la peau et les lèvres. En outre, avec cette dureté, la composition de l'invention résiste bien aux chocs.

Selon l'invention, la composition sous forme de stick a le comportement d'un solide élastique déformable et souple, conférant à l'application une douceur élastique remarquable. Les compositions en stick de l'art antérieur n'ont pas cette propriété d'élasticité et de souplesse.

Le taux du polymère est choisi selon la dureté de gel désirée et en fonction de l'application particulière envisagée. La quantité de polymère doit être telle qu'elle permette l'obtention d'un stick délitable. En pratique, la quantité de polymère (en matière active) représente de 0,5 à 80% du poids total de la composition et mieux de 5 à 40%.

La composition de l'invention peut comprendre, en outre, tout ingrédient usuellement utilisé dans le domaine concerné, et notamment ceux choisis parmi les colorants solubles dans les polyols ou dans la phase grasse, les antioxydants, les huiles essentielles, les conservateurs, les parfums, les polymères liposolubles notamment hydrocarbonés tels que les polyalkylènes ou le polylaurate de vinyle, les gélifiants de phase grasse liquide, les cires, les gommes, les résines, les tensioactifs comme le phosphate de tri-oléyle, les actifs cosmétiques ou dermatologiques additionnels comme par exemple l'eau, les émollients, les hydratants, les vitamines, la lanoline liquide, les acides gras essentiels, les filtres solaires lipophiles ou solubles dans les polyols et leurs mélanges. La composition selon l'invention peut contenir également des vésicules lipidiques de type ionique et/ou non ionique. Ces ingrédients hormis l'eau peuvent être présents dans la composition de façon usuelle à raison de 0 à 20% du poids total de la composition et mieux de 0,1 à 10%.

Au cas où la composition contiendrait une phase aqueuse, ce qui est le cas pour une émulsion simple eau-dans-huile ou huile-dans-eau ou multiple eau-dans-huile-dans-eau ou huile-dans-eau-dans-huile, cette phase aqueuse peut représenter 0,1 à 70% en poids de la composition, notamment de 0,5 à 40% et mieux de 1 à 20%. Cette phase aqueuse peut contenir de l'eau et tout composé miscible à l'eau comme les polyols. Cette phase aqueuse peut, en outre, être gélifiée par des gélifiants appropriés. De préférence, la composition de l'invention se présente sous forme d'une phase continue grasse et plus spécialement sous forme anhydre.

Bien entendu, l'homme du métier veillera à choisir les éventuels ingrédients complémentaires et/ou leur quantité de telle manière que les propriétés avantageuses de la composition selon l'invention ne soient pas ou substantiellement pas, altérées par l'adjonction envisagée.

Les compositions de l'invention peuvent en particulier contenir une ou plusieurs cires, par exemple de la cire de polyéthylène mais on évite l'emploi de cire si l'on veut obtenir des produits brillants. Généralement, la quantité de cire ne dépasse pas 20%, de préférence 10% ; elle représente par exemple de 3 à 5% du poids total de la composition.

La composition selon l'invention peut se présenter sous la forme d'une composition dermatologique ou de soin éventuellement teintée des matières kératiniques comme la peau, les lèvres et/ou les phanères, sous forme d'une composition de protection solaire ou d'hygiène corporelle notamment sous forme de produit démaquillant sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré de maquillage de la peau, en particulier un fond de teint, présentant éventuellement des propriétés de soin ou de traitement, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils en particulier sous forme d'un mascara pain, les sourcils et les cheveux notamment sous forme de crayon. En particulier, la composition de l'invention peut être un produit cosmétique contenant des actifs cosmétiques et/ou dermatologiques comme les hydratants, les céramides, les vitamines, les filtres solaires, les agents cicatrisants.

Dans le cas des compositions de maquillage, des particules solides hydrophobes ou hydrophiles peuvent constituer le(s) pigment(s) permettant de maquiller la peau, les lèvres et/ou les phanères.

Bien entendu, la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur et de toucher et éventuellement de goût agréables.

Par ailleurs, les compositions de maquillage ou de soin conformes à l'invention doivent comporter au moins 10% en masse d'une huile non volatile (siliconée ou non siliconée) et/ou d'un corps gras pâteux, à savoir un produit visqueux contenant une fraction liquide et une fraction solide.

Par "corps gras pâteux" au sens de l'invention, on entend des corps gras ayant un point de fusion allant de 20 à 55°C, de préférence 25 à 45°C, et/ou une viscosité à 40°C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé.

Les valeurs de point de fusion correspondent, selon l'invention, au pic de fusion mesurée par la méthode "Differential Scanning Calorimetry" avec une montée en température de 5 ou 10°C/min.

Selon l'invention, on peut utiliser un ou plusieurs corps gras pâteux. De préférence, ces corps gras sont des composés hydrocarbonés (contenant principalement des atomes de carbone et d'hydrogène et éventuellement des groupements ester), éventuellement de type polymérique ; ils peuvent également être choisis parmi les composés siliconés et/ou fluorés ; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylénées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40°C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le "THIXTNR" de Rheox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stéaryl diméthicones notamment ceux vendus par la Société Dow Corning sous les noms commerciaux de DC2503 et DC25514, et leurs mélanges.

Le ou les corps gras pâteux peuvent être présents à raison de 0,5 à 60% en poids, par rapport au poids total de la composition, de préférence à raison de 2-45% en poids et encore plus préférentiellement à raison de 5-30% en poids dans la composition.

Selon l'invention, la composition peut se présenter aussi sous la forme d'un gel rigide anhydre transparent en l'absence de particules diffusantes comme certaines charges et pigments, et notamment de stick anhydre transparent.

Selon l'invention, la composition peut contenir de plus une matière colorante qui peut être choisie parmi les colorants lipophiles, les colorants hydrophiles, et leurs mélanges.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine, le rocou. Ils peuvent représenter de 0 à 20% du poids de la composition et mieux de 0,1 à 6% (si présents).

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le(s) huile(s), si nécessaire le ou les composés amphiphiles, les matières colorantes et/ou les particules solides, et les additifs, puis à mélanger le tout jusqu'à l'obtention d'une solution homogène à l'oeil nu. Le mélange homogène obtenu peut alors être coulé dans un moule approprié comme un moule de rouge à lèvres ou directement dans les articles de conditionnement (boîtier ou coupelle notamment).

L'invention a encore pour objet un procédé cosmétique pour limiter la migration d'une composition cosmétique contenant une phase grasse liquide comprenant au moins une huile siliconée, consistant à structurer ladite phase grasse par une quantité suffisante d'au moins un polymère (homopolymère ou copolymère) de masse moléculaire moyenne en poids allant de 500 à 500 000, comportant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
la phase grasse liquide étant constituée partiellement ou totalement d'huile(s) volatile(s) ayant un point éclair égal ou supérieur à 40°C et supérieur à la température de ramollissement du polymère gélifiant, à condition que la composition comprenne au moins 10% en masse d'une huile non volatile et/ou d'un produit pâteux ou visqueux.

L'invention est illustrée plus en détail dans les exemples suivants de formulation de maquillage contenant un polyamide siliconé et des pigments et/ou des charges à surface hydrophobe. Les quantités sont données en % massique. Les composés chimiques sont donnés principalement en nom CTFA ("International Cosmetic Ingredient Dictionary"). Les viscosités indiquées sont mesurées à 25°C, à la pression atmosphérique.

### Exemple 1 : Rouge à lèvres

| COMPOSITION | |
|---|---|
| PDMS-DC 200 (5 cSt) | qsp 100% |
| Cyclohexasiloxane D6 | 20% |
| Phényltriméthicone (DC 556 de chez Dow Corning, de 20 cSt) | 12% |
| Isoparaffine hydrogénée (Parleam^{®} de chez Nippon Oil Fats) | 5% |
| Pigments traités hydrophobes (oxydes de fer rouge, jaune et oxyde de titane, | |
| traités au perfluoroalkyle phosphate) | 10% |
| Cire de polyéthylène (Performalen^{®} 500 de chez Petrolite) | 12% |
| Polyamide siliconé de l'exemple 1 du brevet US 5 981 680 | 15% |
| Conservateur qs | |
| Parfum qs | |

Les pigments présentent les indices de couleur (CI) suivants :
oxyde de fer rouge CI : 77491
oxyde de fer jaune CI : 77492
oxyde de titane CI : 77891

Ce rouge à lèvres est obtenu en chauffant la cire et le polymère jusqu'à fusion de l'ensemble puis ajout du Parleam et d'une partie de la phényltriméthicone. Parallèlement, on mélange les pigments et l'autre partie de la phényltriméthicone à température ambiante puis on les passe au broyeur tricylindre. On ajoute ce broyat au mélange fondu de cire et d'huiles de silicone, puis on homogénéise l'ensemble. On refroidit l'ensemble d'au moins 20°C par rapport à la température de fusion du mélange et on ajoute alors le cyclohexasiloxane D6 et le polydiméthylsiloxane 5 cSt, puis le conservateur et le parfum toujours sous agitation. On coule ensuite le mélange dans un moule approprié.

Le produit ainsi obtenu présente des propriétés de tenue, en particulier de la couleur, de glissant et de non gras.

### Exemple 2 : Rouge à lèvres

| COMPOSITION | |
|---|---|
| PDMS-DC 200 (3 cSt) | qsp 100% |
| PDMS oxyéthyléné/oxypropyléné en α-ω dans le cyclopentasiloxane D5 | 3% |
| (Abil EM 90 de chez Golsdschmitd) | |
| Phényltriméthicone (DC 556 de chez Dow Corning, de 20 cSt) | 12% |
| Isoparaffine hydrogénée (Parleam^{®} de chez Nippon Oil Fats) | 5% |
| Pigments (oxydes de fer rouge, jaune, brun et oxyde de titane) | 10% |
| Cire de polyéthylène (Performalen^{®} 500 de chez Petrolite) | 12% |
| Polyamide siliconé de l'exemple 1 du brevet US 5 981 680 | 15% |
| Conservateur qs | |
| Parfum qs | |

Les pigments présentent les indices de couleur (CI) suivants :
oxyde de fer rouge CI : 77491
oxyde de fer jaune CI : 77492
oxyde de fer brun CI : 77491
oxyde de titane CI : 77891

Ce rouge à lèvres est obtenu en chauffant la cire et le polymère jusqu'à fusion de l'ensemble puis ajout du Parleam et d'une partie de la phényltriméthicone. Parallèlement, on mélange les pigments, l'Abil EM 90 et l'autre partie de la phényltriméthicone à température ambiante puis on les passe au broyeur tricylindre. On ajoute ce broyat au mélange fondu de cire et d'huiles de silicone, puis on homogénéise l'ensemble. On refroidit l'ensemble d'au moins 20°C par rapport à la température de fusion du mélange et on ajoute alors le polydiméthylsiloxane 3 cSt, puis le conservateur et le parfum toujours sous agitation. On coule ensuite le mélange dans un moule approprié.

Le produit ainsi obtenu présente des propriétés de tenue, en particulier de la couleur, de glissant et de non gras.

Dans les exemples 1 et 2, le polyamide siliconé utilisé comporte 30 motifs [Si(CH₃)₂-O]. Lorsqu'on utilise à la place de ce polyamide siliconé un polyamide siliconé comportant moins de motifs [Si (CH₃)₂-O], on obtient un gel plus mou et moins brillant. En effet, lorsqu'on augmente le nombre de motifs [Si(CH₃)₂-O] du polymère, on obtient un gel plus dur et brillant.

### Exemple 3 : fond de teint anhydre

| COMPOSITION | |
|---|---|
| PDMS (5 cSt) DC 200 (5 cSt) de Dow Corning | qsp 100% |
| Phényltriméthicone (DC 556) | 12% |
| PDMS (300 cSt) | 5% |
| Pigments traités hydrophobes (oxydes de fer rouge, jaune, brun et oxyde de | |
| titane, traités perfluoroalkyle phosphate) | 10% |
| Cire de polyéthylène (Performalen^{®} 500) | 15% |
| Polyamide siliconé de l'exemple 2 du brevet US 5 981680 | 12% |
| Silice traitée hydrophobe (traitement triméthysiloxyl) | 3% |
| Isononanoate d'isononyle | 10% |
| Isohexadécane | 10% |
| Conservateur qs | |
| Parfum qs | |

Ce fond de teint est préparé comme le rouge à lèvres de l'exemple 1, la silice étant introduite en même temps que la phényltriméthicone dans le broyat pigmentaire, l'isononanoate d'isononyle étant introduit dans le mélanges de cire et d'huiles de silicone et l'isohexadécane en même temps que le cyclohexasiloxane.

Il présente des propriétés de non gras, de glissant, de matité et de bonne tenue dans le temps en particulier de la couleur.

Les particules utilisées sont des particules hydrophobes (ou mieux lipophiles).

### REFERENCES CITEES

**[1]** EP-A-1 068 856
**[2]** US-A-5,874,069
**[3]** US-A-5,919,441
**[4]** US-A-6,051,216
**[5]** US-A-5,981,680
**[6]** US-A-5,412,004
**[7]** EP-A-1,048,686
**[8]** WO-A-02/17870
**[9]** WO-A-02/17871

## Revendications

1. Utilisation d'une phase grasse liquide continue, comprenant au moins une huile siliconée, structurée essentiellement par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
la phase grasse liquide étant constituée partiellement ou totalement d'huile(s) volatile(s) ayant un point éclair égal ou supérieur à 40°C et supérieur à la température de ramollissement du polymère gélifiant,
dans une composition cosmétique de maquillage comprenant des particules solides choisies parmi les charges, les pigments et/ou les nacres et leurs mélanges, ou pour la fabrication d'une composition de maquillage comprenant des particules solides choisies parmi les charges, les pigments et/ou les nacres et leurs mélanges, ladite composition étant physiologiquement acceptable, comme agent pour limiter la migration de ladite composition, à condition que la composition comprenne au moins 10% en masse d'une huile non volatile et/ou d'un produit pâteux ou visqueux.

2. Utilisation selon la revendication 1, dans laquelle la phase grasse est un mélange d'huiles comprenant au moins une huile siliconée non volatile et au moins une huile volatile ayant un point éclair égal ou supérieur à 40°C et supérieur à la température de ramollissement du polymère gélifiant.

3. Utilisation selon la revendication 2, dans laquelle la phase grasse liquide comprend au moins une huile non siliconée, non volatile.

4. Utilisation selon la revendication 1, dans laquelle la phase grasse est constituée uniquement d'huile(s) siliconée(s) volatile(s) ayant un point éclair égal ou supérieur à 40°C et supérieur à la température de ramollissement du polymère gélifiant.

5. Utilisation selon la revendication 2 ou 3, dans laquelle l'huile volatile est une huile siliconée.

6. Utilisation selon la revendication 2 ou 3, dans laquelle l'huile volatile est une huile non siliconée.

7. Utilisation selon la revendication 2 ou 3, dans laquelle le mélange comprend au moins une huile siliconée volatile et au moins une huile non siliconée volatile ayant des points éclairs égaux ou supérieurs à 40°C et supérieurs à la température de ramollissement du polymère gélifiant.

8. Utilisation selon l'une quelconque des revendications 4, 5 et 7 dans laquelle l'huile siliconée volatile est choisie dans le groupe constitué par les composés suivants : l'octyltriméthicone, l'hexyltriméthicone, l'octaméthyl cyclotétratasiloxane D4, le dodécaméthyl cyclohexasiloxane D6, l'heptaméthyl octyltrisiloxane, le décaméthyl tétrasiloxane, le dodécaméthyl pentasiloxane, le polydiméthylsiloxane de 1,5 cSt, le polydiméthylsiloxane de 2 cSt, le polyméthylsiloxane de 3 cSt, le polydiméthylsiloxane de 5 cSt, et leurs mélanges.

9. Utilisation selon l'une quelconque des revendications 6 et 7, dans laquelle l'huile volatile non siliconée est choisie dans le groupe constitué par les composés suivants : l'isododécane, l'isohexadécane, les isoparaffines en C₈-C₁₆, le néopentanoate d'isodécyle, le n-butyl éther de propylèneglycol, le 3-éthoxypropionate d'éthyle, l'acétate de méthyléther de propylèneglycol, le néopentanoate-d'isohéxyle et leurs mélanges.

10. Utilisation selon l'une quelconque des revendications précédentes, **caractérisée en ce que** l'huile volatile présente un point éclair supérieur à 60°C, de préférence supérieur à 80°C et mieux encore supérieur à 93°C.

11. Utilisation selon l'une quelconque des revendications précédentes, **caractérisée en ce que** l'huile volatile présente un point éclair égal ou inférieur à 160°C, de préférence égal ou inférieur à 135°C.

12. Utilisation selon l'une quelconque des revendications précédentes, dans laquelle la phase grasse liquide contient au moins 40% et mieux encore au moins 50% en poids d'huile siliconée.

13. Utilisation selon l'une quelconque des revendications précédentes, dans laquelle l'huile volatile représente de 3 à 89,4% du poids total de la composition.

14. Utilisation selon l'une quelconque des revendications précédentes, comprenant en outre une matière colorante.

15. Utilisation selon la revendication 1, dans laquelle les particules solides sont des particules hydrophobes.

16. Utilisation selon la revendication 1, dans laquelle les particules solides sont des particules hydrophiles, enrobées dans un film de composé hydrophobe.

17. Utilisation selon la revendication 1, dans laquelle les particules solides sont des particules hydrophiles et la composition comprend de plus une silicone amphiphile.

18. Utilisation selon la revendication 1, dans laquelle les particules sont des pigments choisis parmi les oxydes de zinc, les oxydes de fer et les oxydes de titane et leurs mélanges.

19. Utilisation selon l'une quelconque des revendications 1 à 18, dans laquelle le polymère utilisé comme agent gélifiant comprend au moins un motif répondant à la formule : 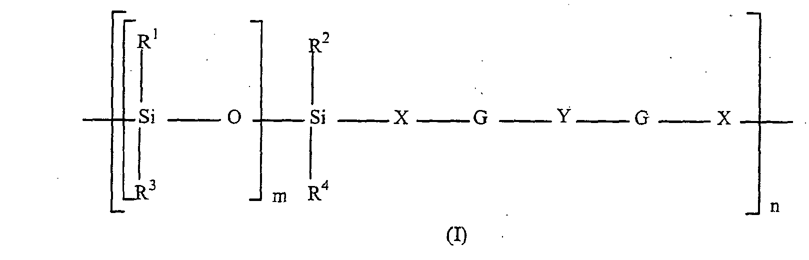 dans laquelle :
1) R¹, R², R³ et R⁴, identiques ou différents, représentent un groupe choisi parmi :
- les groupes hydrocarbonés, linéaires, ramifiésou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en C6 à C10, éventuellement substitués par un ou plusieurs groupes alkyle en C1 à C4,
- les chaînes polyorganosiloxanes contenant ou non un ou plusieurs atomes d'oxygène, de soufre et/ou d'azote ;
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote ;
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C1 à C6, ou
4) Y représente un groupe répondant à la formule : 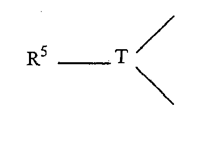 dans laquelle
- T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
- R⁵ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 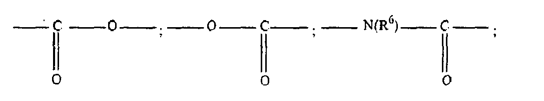 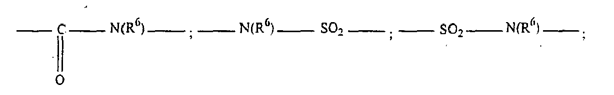 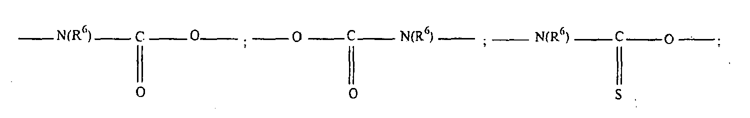 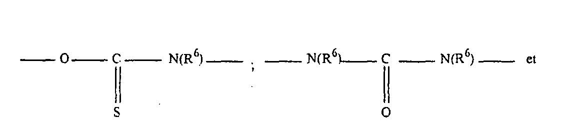 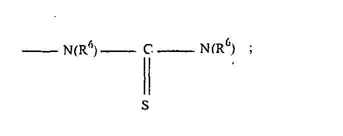 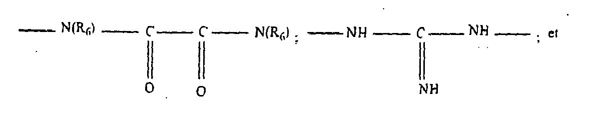 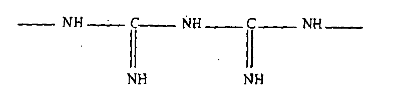 où R⁶ représente un atome d'hydrogène ou un groupe alkyle, linéaire ou ramifié, en C₁ à C₂₀, à condition qu'au moins 50% des R⁶ du polymère représente un atome d'hydrogène et qu'au moins deux des groupes G du polymère soient un autre groupe que : 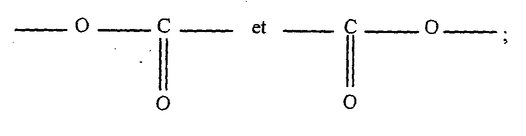
6) n est un nombre entier allant de 2 à 500, de préférence de 2 à 200, et m est un nombre entier allant de 1 à 1000, de préférence de 1 à 700 et mieux encore de 6 à 200.

20. Utilisation selon la revendication 19, dans laquelle Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5_groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆, et
g) les chaînes polyorganosiloxane de formule : 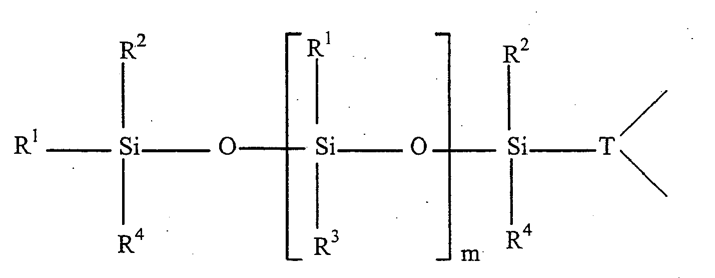
dans laquelle R¹, R², R³, R⁴, T et m sont tels que définis ci-dessus.

21. Utilisation selon l'une quelconque des revendications 1 à 18, dans laquelle le polymère utilisé comme agent gélifiant comprend au moins un motif répondant à la formule (II) : 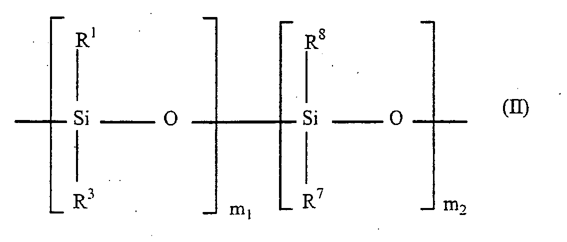 dans laquelle
- R¹ et R³, identiques ou différents, sont tels que définis ci-dessus pour la formule (I) dans la revendication 19,
- R⁷ représente un groupe tel que défini ci-dessus pour R¹ et R³, ou représente le groupe de formule -X-G-R⁹ dans laquelle X et G sont tels que définis ci-dessus pour la formule (I) dans la revendication 19, et R⁹ représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
- R⁸ représente le groupe de formule -X-G-R⁹ dans laquelle X, G et R⁹ sont tels que définis ci-dessus,
- m₁ est un nombre entier allant de 1 à 998, et
- m₂ est un nombre entier allant de 2 à 500.

22. Utilisation selon la revendication 19, dans laquelle le polymère comprend au moins un motif de formule (III) ou (IV) : 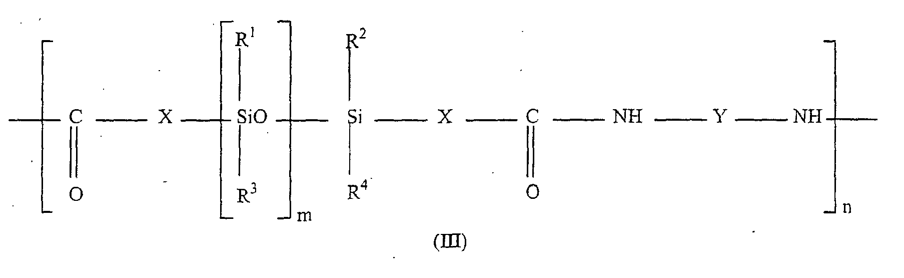 ou 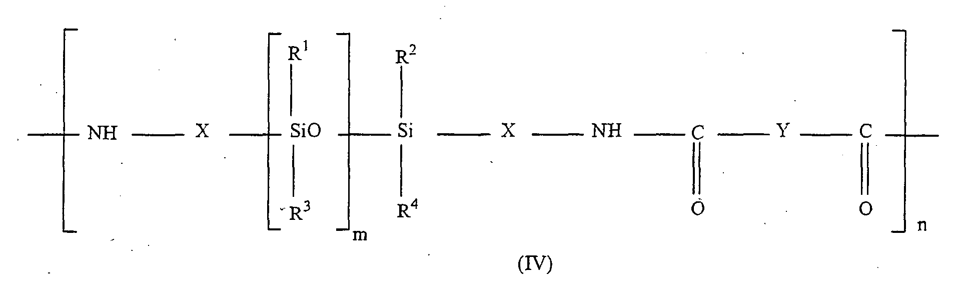 dans lesquelles R¹, R², R³, R⁴, X, Y, m et n sont tels que définis dans la revendication 19.

23. Utilisation selon l'une quelconque des revendications 19 et 20, dans laquelle X et/ou Y représentent un groupe alkylène contenant dans sa partie alkylène au moins l'un des éléments suivants :
1°) 1 à 5 groupes amides, urée ou carbamate,
2°) un groupe cycloalkyle en C₅ ou C₆, et
3°) un groupe phénylène éventuellement substitué par 1 à 3 groupes alkyles identiques ou différents en C₁ à C₃, et/ou substitué par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

24. Utilisation selon l'une quelconque des revendications 19 à 22, dans laquelle Y représente : 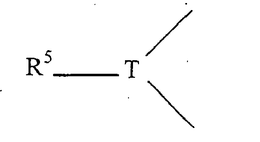 où R⁵ représente une chaîne polyorganosiloxane, et T représente un groupe de formule : 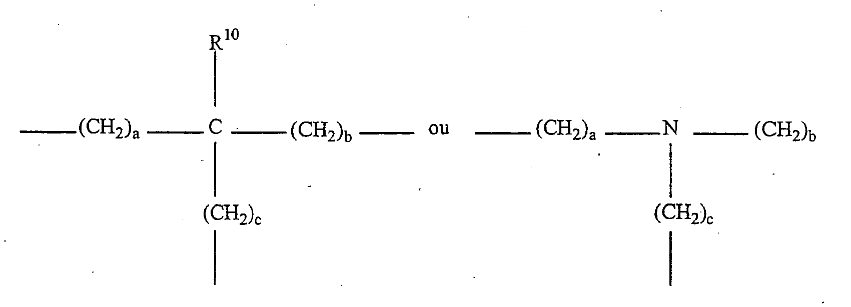 dans lesquelles a, b et c sont indépendamment des nombres entiers allant de 1 à 10, et R¹⁰ est un atome d'hydrogène ou un groupe tel que ceux définis pour R¹, R², R³ et R⁴, dans la revendication 19.

25. Utilisation selon l'une quelconque des revendications 19 à 22, dans laquelle R¹, R², R³ et R⁴ représentent, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

26. Utilisation selon la revendication 1, dans laquelle le polymère utilisé comme agent gélifiant comprend au moins un motif de formule : 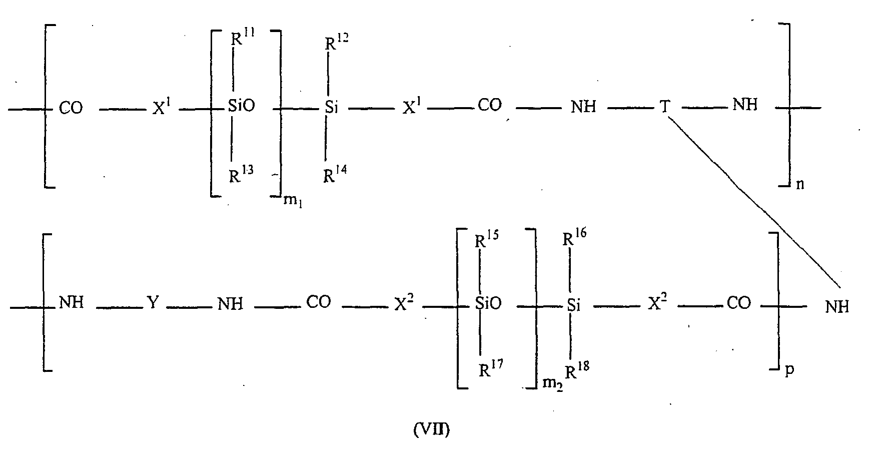 dans laquelle X¹ et X² qui sont identiques ou différents, ont la signification donnée pour X dans la revendication 19, n, Y et T sont tels que définis dans la revendication 19, R¹¹ à R¹⁸ sont des groupes choisis dans le même groupe que les R¹ à R⁴ de la revendication 19, m₁ et m₂ sont des nombres situés dans la gamme allant de 1 à 1 000, et p est un nombre entier allant de 2 à 500.

27. Utilisation selon la revendication 26, dans laquelle :
- p est dans la gamme de 1 à 25, mieux encore de 1 à 7,
- R¹¹ à R¹⁸ sont des groupes méthyle,
- T répond à l'une des formules suivantes : 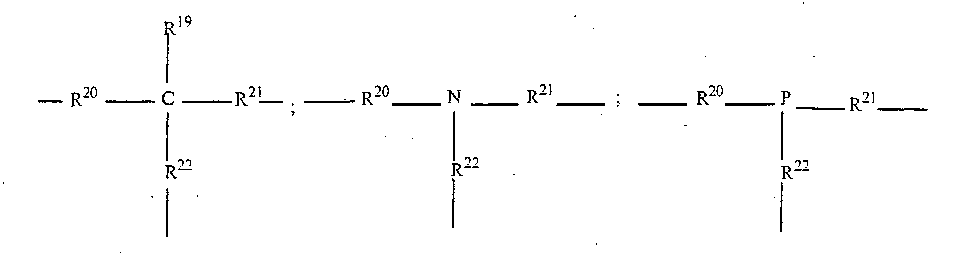 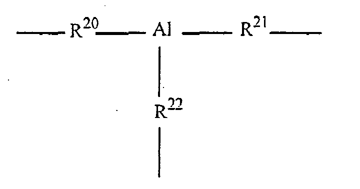
dans lesquelles R¹⁹ est un atome d'hydrogène ou un groupe choisi parmi les groupes définis pour R¹ à R⁴, et R²⁰, R²¹ et R²² sont indépendamment des groupes alkylène, linéaires ou ramifiés, de préférence encore, à la formule : 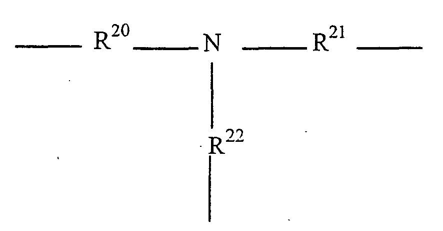 en particulier avec R²⁰, R²¹ et R²² représentant -CH₂-CH₂-,
- m₁ et m₂ sont dans la gamme de 15 à 500, et mieux encore de 15 à 45,
- X¹ et X² représentent - (CH₂)₁₀-, et
- Y représente -CH₂-.

28. Utilisation selon l'une quelconque des revendications 1 à 18, dans laquelle le polymère comprend au moins un motif répondant à la formule suivante : 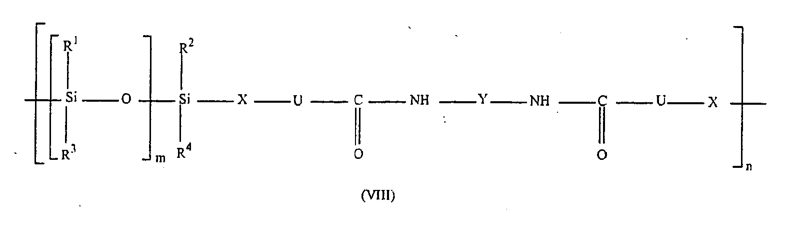 dans laquelle les R¹, R², R³, R⁴, X, Y, m et n ont les significations données ci-dessus pour la formule (I) dans la revendication 19, et U représente -O- ou -NH-, ou
Y représente un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane, ou Y représente un radical alkylène en C₁ à C₄₀, linéaire ou ramifié, ou un radical cycloalkylène en C₄ à C₁₂, ou
Y représente une séquence polyuréthane ou polyurée correspondant à la condensation de plusieurs molécules de diisocyanate avec une ou plusieurs molécules de coupleurs du type diol ou diamine, répondant à la formule : 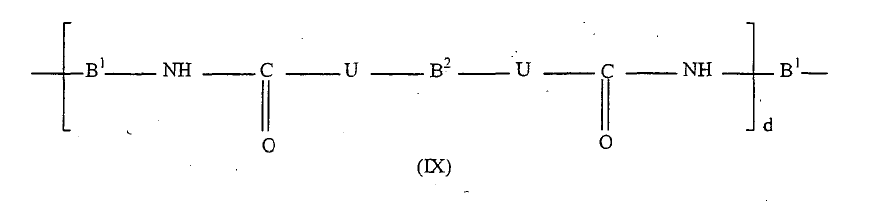
dans laquelle B¹ est un groupe choisi parmi les groupes donnés ci-dessus pour Y, U est -O- ou -NH-, et B² est choisi parmi :
• les groupes alkylène en C₁ à C₄₀, linéaires ou ramifiés, qui peuvent porter éventuellement un groupe ionisable tel qu'un groupe acide carboxylique ou sulfonique, ou un groupe amine tertiaire neutralisable ou quaternisable,
• les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
• les groupes phénylène pouvant éventuellement être porteurs de substituants alkyles en C₁ à C₃, et
• les groupes de formule : 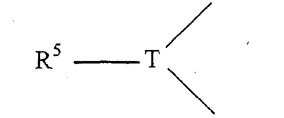
dans laquelle T est un radical trivalent hydrocarboné pouvant contenir un ou plusieurs hétéroatomes tels que l'oxygène, le soufre et l'azote et R⁵ est une chaîne polyorganosiloxane ou une chaîne alkyle en C₁ à C₅₀, linéaire ou ramifiée.

29. Utilisation selon l'une quelconque des revendications 1 à 18, dans laquelle le polymère comprend au moins un motif de formule : 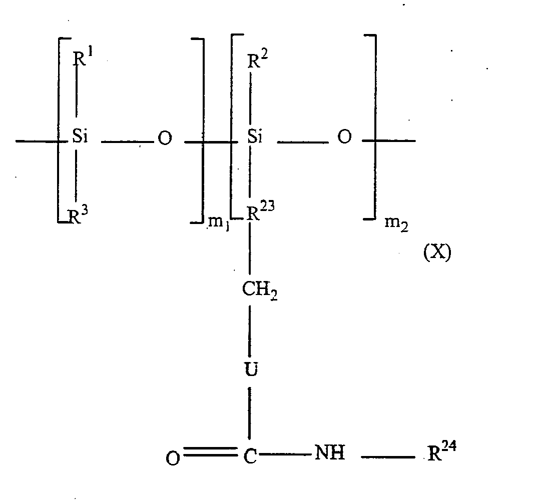 dans laquelle R¹, R², R³, m₁ et m₂ ont les significations données ci-dessus pour la formule (I),
- U représente O ou NH,
- R²³ représente un groupe alkylène en C₁ à C₄₀, comportant éventuellement un ou plusieurs hétéroatomes choisis parmi O et N, ou un groupe phénylène, et
- R²⁴ est choisi parmi les groupes alkyle en C₁ à C₅₀, linéaires, ramifiés ou cycliques, saturés ou insaturés, et les groupes phényle éventuellement substitués par un à trois groupes alkyles en C₁ à C₃.

30. Utilisation selon l'une quelconque des revendications 1 à 18, dans laquelle le polymère utilisé comme agent gélifiant comprend au moins un motif de formule : 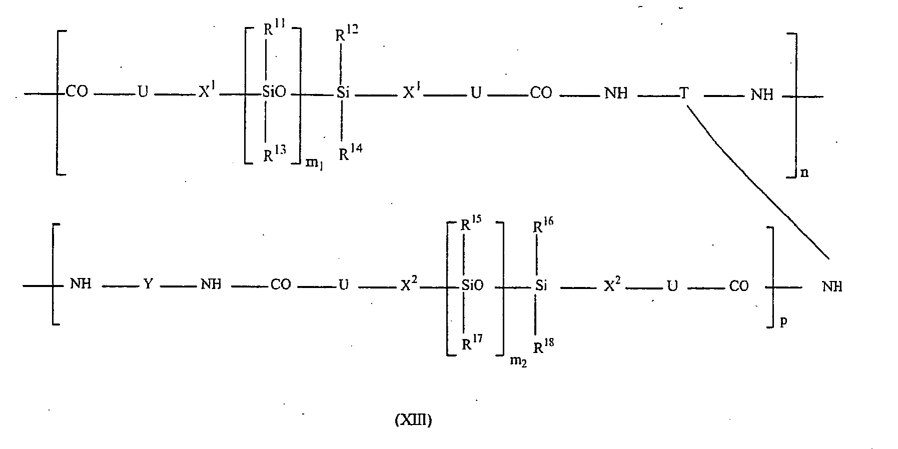 dans laquelle X¹ et X² qui sont identiques ou différents, ont la signification donnée pour X dans la revendication 19, n, Y et T sont tels que définis dans la revendication 19, R¹¹ à R¹⁸ sont des groupes choisis dans le même groupe que les R¹ à R⁴ de la revendication 19, m₁ et m₂ sont des nombres situés dans la gamme allant de 1 à 1 000, et p est un nombre entier allant de 2 à 500.

31. Utilisation selon l'une quelconque des revendications 19 à 30, dans laquelle le polymère utilisé comme agent gélifiant comprend de plus un motif hydrocarboné comportant deux groupes capables d'établir des interactions hydrogène choisis parmi les groupes ester, amide, sulfonamide, carbamate, thiocarbamate, urée, thiourée et leurs combinaisons.

32. Utilisation selon la revendication 30, dans laquelle le copolymère est un copolymère bloc, un copolymère séquencé ou un copolymère greffé.

33. Utilisation selon l'une quelconque des revendications précédentes, dans laquelle le polymère représente de 0,5 à 80% et mieux de 5 à 40% du poids total de la composition.

34. Utilisation selon l'une quelconque des revendications précédentes, dans laquelle le rapport massique polymère gélifiant/huile(s) siliconée(s) est de 0,1 à 50%.

35. Utilisation selon l'une quelconque des revendications précédentes, dans laquelle la phase grasse liquide représente de 5 à 99% du poids total de la composition et mieux de 20 à 75%.

36. Utilisation selon l'une des revendications précédentes, **caractérisée en ce que** la composition contient, en outre, au moins un actif cosmétique ou dermatologique.

37. Utilisation selon l'une quelconque des revendications précédentes, **caractérisée en ce que** la composition elle contient au moins un additif choisi parmi l'eau, les antioxydants, les huiles essentielles, les conservateurs, les parfums, les polymères liposolubles notamment hydrocarbonés tels que les polyalkylènes ou le polylaurate de vinyle, les gélifiants de phase aqueuse, les gélifiants de phase grasse liquide, les cires, les gommes, les résines, les tensioactifs comme le phosphate de tri-oléyle, les actifs cosmétiques ou dermatologiques additionnels choisis dans le groupe constitué par les émollients, les hydratants, les vitamines, la lanoline liquide, les acides gras essentiels, les filtres solaires lipophiles ou solubles dans les polyols et leurs mélanges

38. Utilisation selon l'une quelconque des revendications précédentes, **caractérisée en ce que** la composition se présente sous la forme d'un gel rigide anhydre transparent, et notamment de stick anhydre transparent.

39. Utilisation selon la Rev. 1 dans laquelle la composition est solide structurée de maquillage de la peau, des lèvres et/ou des phanères, contenant au moins un pigment et/ou une nacre en quantité suffisante pour maquiller la peau, les lèvres et/ou les phanères et une phase grasse continue liquide, ladite composition comprenant au moins 10% en masse d'une huile non volatile et/ou d'un produit pâteux ou visqueux, et ladite composition se présentant sous forme d'un solide, et le pigment et/ou la nacre, la phase grasse liquide et le polymère formant un milieu physiologiquement acceptable.

40. Utilisation selon la revendication 39 dans laquelle la composition est une composition structurée de rouge à lèvres, contenant au moins un pigment et/ou une nacre en quantité suffisante pour maquiller les lèvres.

41. Utilisation selon la revendication 38, dans laquelle le polymère comprend au moins un motif répondant à l'une des formules suivantes : formule (I) de la revendication 19, formule (II) de la revendication 21, formules (III) et (IV) de la revendication 22, formule (VII) de la revendication 26, formule (VIII) de la revendication 28, formule (X) de la revendication 29 et formule (XIII) de la revendication 30.

42. Utilisation selon l'une quelconque des revendications 1 à 41, **caractérisé en ce que** la composition se présente sous forme de mascara pain, d'eye-liner, de fond de teint, de rouge à lèvres, de blush, de produit de maquillage du corps, de fard à paupières ou à joues, de produit anti-cerne.

43. Utilisation selon la revendication 1 dans laquelle la composition qui se présente sous la forme d'un stick de maquillage de la peau, des lèvres et/ou des phanères, et en particulier des lèvres, contenant au moins un pigment et/ou une nacre en quantité suffisante pour maquiller la peau, les lèvres et/ou les phanères, la composition comprenant au moins 10% en masse d'une huile non volatile et/ou d'un produit pâteux ou visqueux, le pigment et/ou la nacre, la phase grasse et le polymère formant un milieu physiologiquement acceptable.

44. Utilisation selon la revendication 1, dans laquelle la composition a une dureté de 20 à 2 000 gf, de préférence de 20 à 900 gf et mieux de 20 à 600 gf.

45. Procédé cosmétique pour limiter la migration d'une composition cosmétique de maquillage comprenant des particules solides choisies parmi les charges, les pigments et/ou les nacres et leurs mélanges, ladite composition contenant une phase grasse liquide comprenant au moins une huile siliconée, consistant à structurer ladite phase grasse par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
la phase grasse liquide étant constituée partiellement ou totalement d'huile(s) volatile(s) ayant un point éclair égal ou supérieur à 40°C et supérieur à la température de ramollissement du polymère gélifiant, à condition que la composition comprenne au moins 10% en masse d'une huile non volatile et/ou d'un produit pâteux ou visqueux.

## Claims

1. Use of a continuous, liquid fatty phase comprising at least one silicone oil, essentially structured with an adequate quantity of at least one polymer (homopolymer or copolymer) with a weight-average molecular mass ranging from 500 to 500,000, containing at least one moiety comprising:
- at least one polyorganosiloxane group, consisting of from 1 to 1,000 organosiloxane units in the chain of the moiety or in the form of a graft, and
- at least two groups capable of establishing hydrogen interactions, chosen from ester, amide, sulphonamide, carbamate, thiocarbamate, urea, thiourea, oxamido, guanidino and biguanidino groups, and combinations thereof, on condition that at least one of the groups is other than an ester group,
the polymer being solid at room temperature and soluble in the liquid fatty phase at a temperature of from 25 to 250°C,
the liquid fatty phase consisting partially or totally of volatile oil(s) with a flash point greater than or equal to 40°C and greater than the softening point of the gelling polymer,
in a cosmetic makeup composition comprising solid particles chosen from among fillers, pigments and/or glosses and their mixtures, or for producing a makeup composition comprising solid particles chosen from among fillers, pigments and/or glosses and their mixtures, said composition being physiologically acceptable, as an agent for limiting the migration of said composition, provided that the composition comprises at least 10 wt.% of a non-volatile oil and/or a pasty or viscous product.

2. Use according to claim 1, in which the fatty phase is a mixture of oils comprising at least one non-volatile silicone oil and at least one volatile silicone oil with a flash point greater than or equal to 40°C and greater than the softening point of the gelling polymer.

3. Use according to claims 2, in which the liquid fatty phase comprises at least one non-volatile non-silicone oil.

4. Use according to claim 1, in which the fatty phase consists solely of volatile silicone oil(s) with a flash point greater than or equal to 40°C and greater than the softening point of the gelling polymer.

5. Use according to claim 2 or 3, in which the volatile oil is a silicone oil.

6. Use according to claim 2 or 3, in which the volatile oil is a non-silicone oil.

7. Use according to claim 2 or 3, in which the mixture comprises at least one volatile silicone oil and at least one volatile non-silicone oil with flash points greater than or equal to 40°C and greater than the softening point of the gelling polymer.

8. Use according to any one of claims 4, 5 and 7, in which the volatile silicone oil is chosen from the group consisting of the following compounds: octyltrimethicone, hexyltrimethicone, octamethyl cyclotetrasiloxane D4, dodecamethyl cyclohexasiloxane D6, heptamethyl octyltrisiloxane, decamethyl tetrasiloxane, dodecamethyl pentasiloxane, polydimethylsiloxane of 1.5 cSt, polydimethylsiloxane of 2 cSt, polymethylsiloxane of 3 cSt, polydimethylsiloxane of 5 cSt, and mixtures thereof.

9. Use according to either of claims 6 and 7, in which the non-silicone volatile oil is chosen from the group consisting of the following compounds: isododecane, isohexadecane, C₈-C₁₆ isoparaffins, isodecyl neopentanoate, propylene glycol n-butyl ether, ethyl 3-ethaxypropionate, propylene glycol methyl ether acetate, isohexyl neopentanoate, and mixtures thereof.

10. Use according to any one of the preceding claims, **characterized in that** the volatile oil has a flash point of greater than 60°C, preferably greater than 80°C and better still greater than 93°C.

11. Use according to any one of the preceding claims, **characterized in that** the volatile oil has a flash point of less than or equal to 160°C and preferably less than or equal to 135°C.

12. Use according to any ane of the preceding claims, in which the liquid fatty phase contains at least 40% and better still at least 50% by weight of silicone oil.

13. Use according to any one of the preceding claims, in which the volatile oil represents from 3% to 89.4% of the total weight of the composition.

14. Use according to any one of the preceding claims, also comprising a dyestuff.

15. Use according to claim 1, in which the solid particles are hydrophobic particles.

16. Use according to claim 1, in which the solid particles are hydrophilic particles, coated in a film of hydrophobic compound.

17. Use according to claim 1, in which the solid particles are hydrophilic particles and the composition furthermore comprises an amphiphilic silicone.

18. Use according to one of claim 1, in which the particles are pigments chosen from zinc oxides, iron oxides and titanium oxides, and mixtures thereof.

19. Use according to any one of claims 1 to 18, in which the polymer used as gelling agent comprises at least one moiety corresponding to the formula: 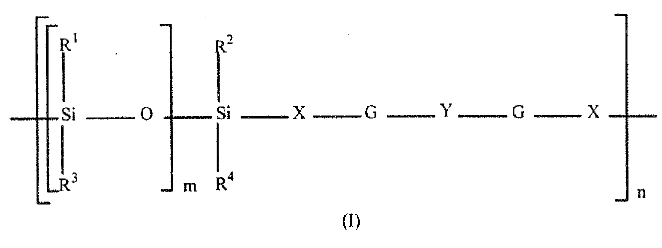 in which:
1) R¹, R², R³ and R⁴, which may be identical or different, represent a group chosen from:
- linear, branched or cyclic, saturated, or unsaturated, C₁ to C₄₀ hydrocarbon-based groups, possibly containing in their chain one or more oxygen, sulphur and/or nitrogen atoms, and possibly being partially or totally substituted with fluorine atoms,
- C₆ to C₁₀ aryl groups, optionally substituted with one or more C₁ to C₄ alkyl groups,
- polyorganosiloxane chains possibly containing one or more oxygen, sulphur and/or nitrogen atoms;
2) the groups X, which may be identical or different, represent a linear or branched C₁ to C₃₀ alkylenediyl group, possibly containing in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituents one of the following atoms or groups of atoms: fluorine, hydroxyl, C₃ to C₈ cycloalkyl, C₁ to C₄₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
4) Y represents a group corresponding to the formula: 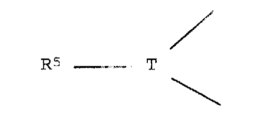 in which
- T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
- R⁵ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
5) the groups G, which may be identical or different, represent divalent groups chosen from: 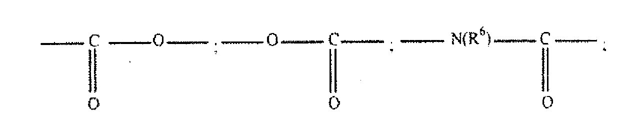 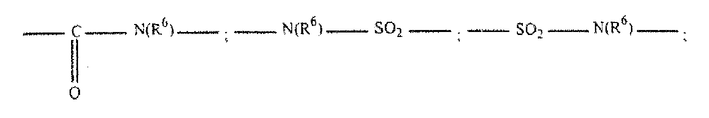 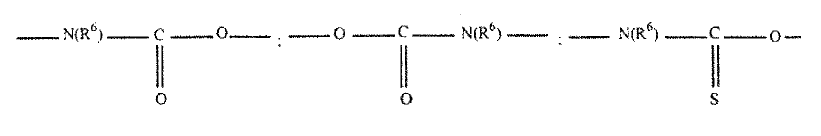 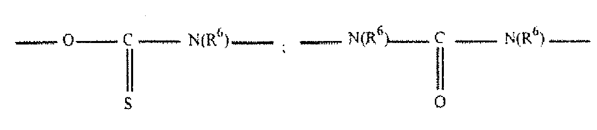 and 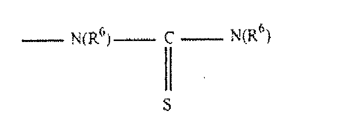 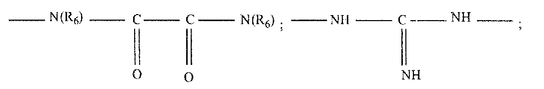 and 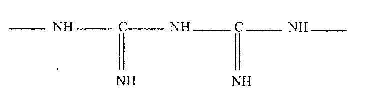 in which R⁶ represents a hydrogen atom or a linear or branched C₁ to C₂₀ alkyl group, on condition that at least 50% of the groups R⁶ of the polymer represents a hydrogen atom and that at least two of the groups G of the polymer are a group other than: 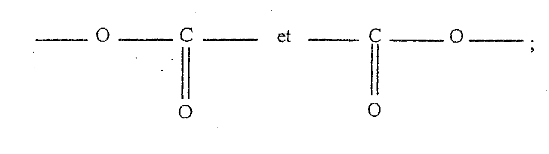
6) n is an integer ranging from 2 to 500 and preferably from 2 to 200, and m is an integer ranging from 1 to 1,000, preferably from 1 to 700 and better still from 6 to 200.

20. Use according to claim 19, in which Y represents a group chosen from among:
a) linear C₁ to C₂₀ and preferably C₁ to C₁₀ linear alkylene groups,
b) branched alkylene groups which can have C₃₀ to C₅₆ non-conjugate unsaturations and cycles,
c) C₅-C₆ cycloalkylene groups,
d) phenylene groups optionally substituted by one or more C₁ to C₄₀ alkyl groups,
e) C₁ to C₂₀ alkylene groups having 1 to 5 amide groups,
f) C₁ to C₂₀ alkylene groups having one or more substituents chosen from among hydroxyl, C₃ to C₈ cycloalkane groups, C₁ to C₃ hydroxyalkyl groups and C₁ to C₆ alkylamine groups and
g) polyorganosiloxane chains of formula: 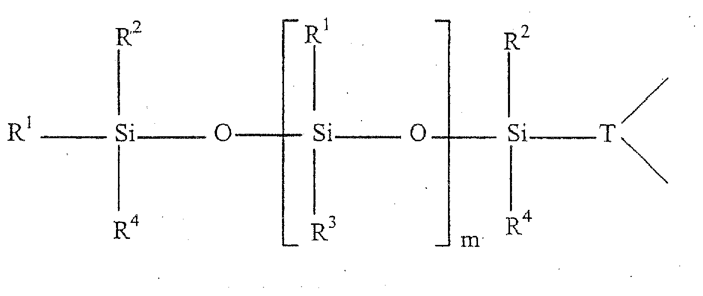
in which R¹, R², R³, R⁴, T and m are as defined hereinbefore.

21. Use according to any one of claims 1 to 18, in which the polymer used as gelling agent comprises at least one moiety corresponding to formula (II): 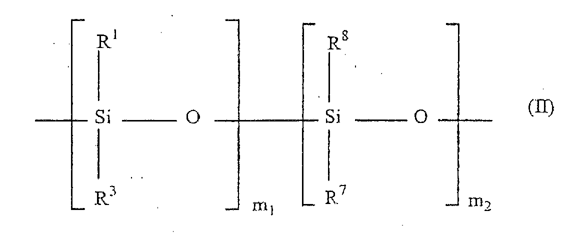 in which
- R¹ and R³, which may be identical or different, are as defined above for formula (I) in claim 19,
- R⁷ represents a group as defined above for R¹ and R³, or represents a group of formula -X-G-R⁹ in which X and G are as defined above for formula (I) in claim 19, and R⁹ represents a hydrogen atom or a linear, branched or cyclic, saturated or unsaturated, C₁ to C₅₀ hydrocarbon-based group optionally comprising in its chain one or more atoms chosen from O, S and N, optionally substituted with one or more fluorine atoms and/or one or more hydroxyl groups, or a phenyl group optionally substituted with one or more C₁ to C₄ alkyl groups,
- R⁸ represents a group of formula -X-G-R⁹ in which X, G and R⁹ are as defined above,
- m₁ is an integer ranging from 1 to 998, and
- m₂ is an integer ranging from 2 to 500.

22. Use according to claim 19, in which the polymer comprises at least one moiety of formula (III) or (IV): 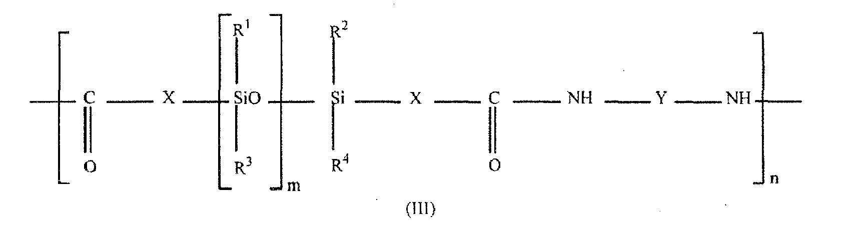 or 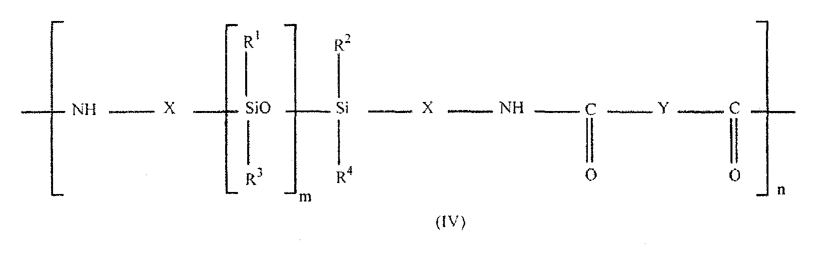 in which R¹, R², R³, R⁴, X, Y, m and n are as defined in claim 19.

23. Use according to either of claims 19 and 20, in which X and/or Y represents an alkylene group containing in its alkylene portion at least one of the following elements:
1°) 1 to 5 amide, urea or carbamate groups,
2°) a C₅ or C₆ cycloalkyl group, and
3°) a phenylene group optionally substituted with 1 to 3 identical or different C₁ to C₃ alkyl groups, and/or substituted with at least one element chosen from the group consisting of:
- a hydroxyl group,
- a C₃ to C₈ cycloalkyl group,
- one to three C₁ to C₄₀ alkyl groups,
- a phenyl group optionally substituted with one to three C₁ to C₃ alkyl groups,
- a C₁ to C₃ hydroxyalkyl group, and
- a C₁ to C₆ aminoalkyl group.

24. Use according to any one of claims 19 to 22, in which Y represents: 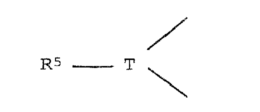 in which R⁵ represents a polyorganosiloxane chain and T represents a group of formula: 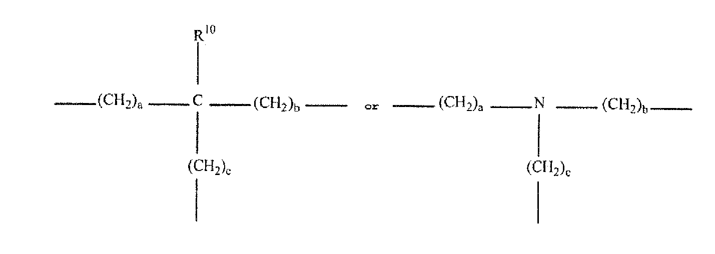 in which a, b and c are, independently integers ranging from 1 to 10, and R¹⁰ is a hydrogen atom or a group such as those defined for R¹, R², R³ and R⁴, in claim 19.

25. Use according to any one of claims 19 to 22, in which R¹, R², R³ and R⁴ represent, independently, a linear or branched C₁ to C₄₀ alkyl group, preferably a CH₃, C₂H₅, n-C₃H₇ or isopropyl group, a polyorganosiloxane chain or a phenyl group optionally substituted with one to three methyl or ethyl groups.

26. Use according to claim 1, in which the polymer used as gelling agent comprises at least one moiety of formula: 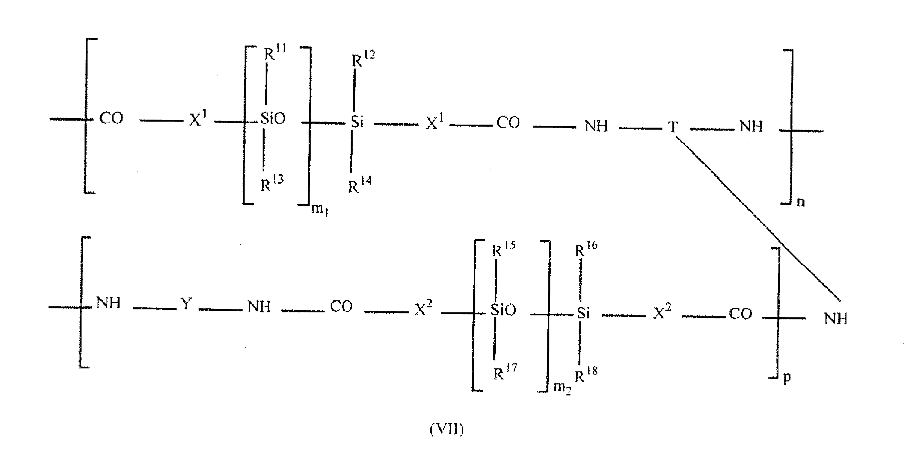 in which X¹ and X², which may be identical or different, have the meaning given for X in claim 19, n, Y and T are as defined in claim 19, R¹¹ to R¹⁸ are groups chosen from the same group as R¹ to R⁴ of claim 19, m₁ and m₂ are numbers in the range from 1 to 1,000, and p is an integer ranging from 2 to 500.

27. Composition according to claim 26, in which:
- p is in the range from 1 to 25 and better still from 1 to 7,
- R¹¹ to R¹⁸ are methyl groups,
- T corresponds to one of the following formulae: 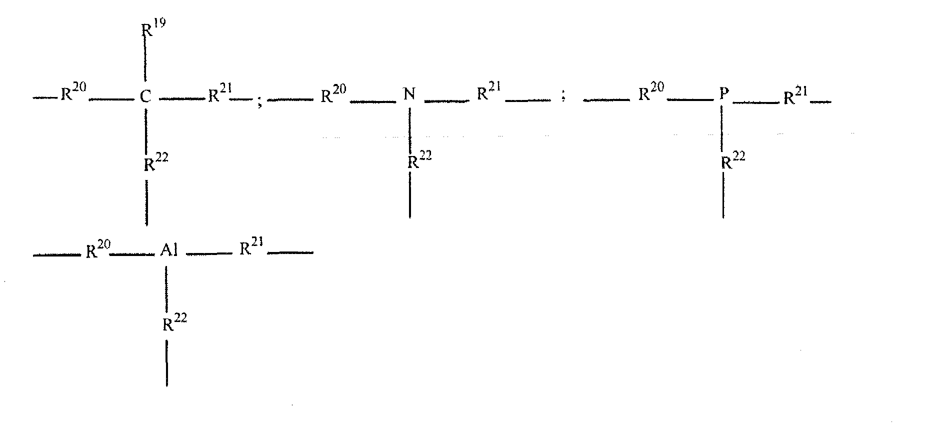
in which R¹⁹ is a hydrogen atom or a group chosen from the groups defined for R¹ to R⁴, and R²⁰, R²¹ and R²² are, independently linear or branched alkylene groups, and more preferably corresponds to the formula: 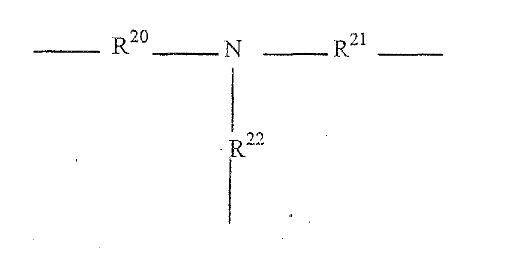 in particular with R²⁰, R²¹ and R²² representing -CH₂-CH₂-,
- m₁ and m₂ are in the range from 15 to 500 and better still from 15 to 45,
- X¹ and X² represent -(CH₂)₁₀-, and
- Y represents -CH₂-.

28. Use according to any one of claims 1 to 18, in which the polymer comprises at least one moiety corresponding to the following formula: 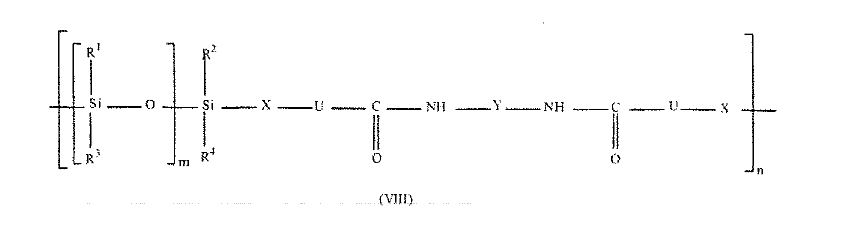 in which R¹, R², R³, R⁴, X, Y, m and n have the meanings given above for formula (I) in claim 19, and U represents -O- or -NH-, or
Y represents a C₅ to C₁₂ cycloaliphatic or aromatic group that may be substituted with a C₁ to C₁₅ alkyl group or a C₅ to C₁₀ aryl group, for example a radical chosen from the methylene-4,4-biscyclohexyl radical, the radical derived from isophorone diisocyanate, 2,4- and 2,6-tolylenes, 1,5-naphthylene, p-phenylene and 4,4'-biphenylenemethane or Y represents a linear or branched C₁ to C₄₀ alkylene radical or a C₄ to C₁₂ cycloalkylene radical, or
Y represents a polyurethane or polyurea block corresponding to the condensation of several diisocyanate molecules with one or more coupling agents of the diol or diamine type, corresponding to the formula: 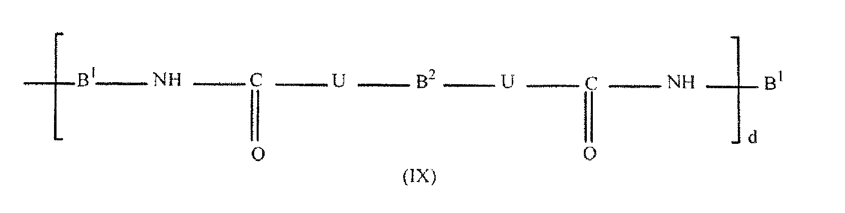 in which B¹ is a group chosen from the groups given above for Y, U is -O- or -NH- and B² is chosen from:
• linear or branched C₁ to C₄₀ alkylene groups, which can optionally bear an ionizable group such as a carboxylic acid or sulphonic acid group, or a neutralizable or quaternizable tertiary amine group,
• C₅ to C₁₂ cycloalkylene groups, optionally bearing alkyl substituents, for example one to three methyl or ethyl groups, or alkylene, for example the diol radical: cyclohexanedimethanol,
• phenylene groups that may optionally bear C₁ to C₃ alkyl substituents, and
• groups of formula: 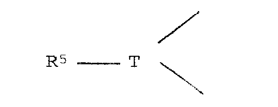
in which T is a hydrocarbon-based trivalent radical possibly containing one or more hetero atoms such as oxygen, sulphur and nitrogen and R⁵ is a polyorganosiloxane chain or a linear or branched C₁ to C₅₀ alkyl chain.

29. Use according to any one of claims 1 to 18, in which the polymer comprises at least one moiety of formula: 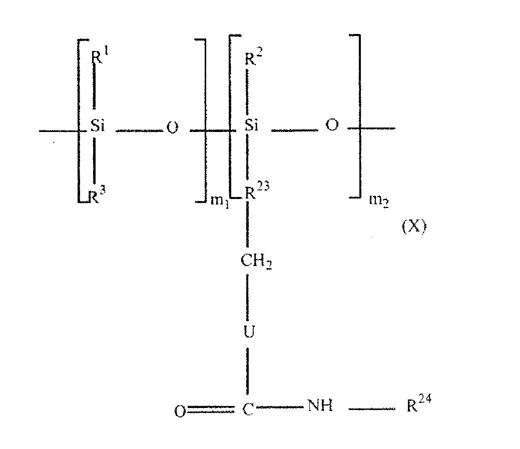 in which R¹, R², R³, m₁ and m₂ have the meanings given above for formula (I),
- U represents O or NH,
- R²³ represents a C₁ to C₄₀ alkylene group, optionally comprising one or more hetero atoms chosen from O and N, or a phenylene group, and
- R²⁴ is chosen from linear, branched or cyclic, saturated or unsaturated C₁ to C₅₀ alkyl groups, and phenyl groups optionally substituted with one to three C₁ to C₃ alkyl groups.

30. Use according to any one of claims 1 to 18, in which the polymer used as gelling agent comprises at least one moiety of formula: 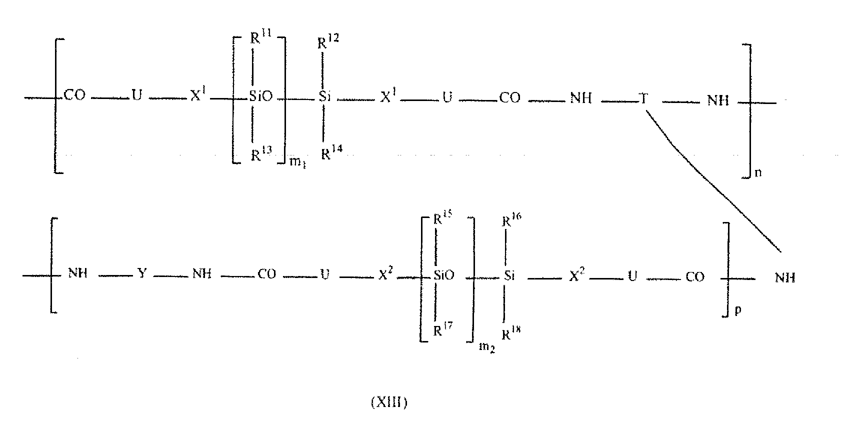 in which X¹ and X², which are identical or different, have the meaning given for X in claim 19, n, Y and T are as defined in claim 19, R¹¹ to R¹⁸ are groups chosen from the same group as R¹ to R⁴ of claim 19, m₁ and m₂ are numbers in the range from 1 to 1,000, and p is an integer ranging from 2 to 500.

31. Use according to any one of claims 19 to 30, in which the polymer used as gelling agent furthermore comprises a hydrocarbon-based moiety comprising two groups capable of establishing hydrogen interactions, chosen from ester, amide, sulphonamide, carbamate, thiocarbamate, urea and thiourea groups, and combinations thereof.

32. Use according to claim 30, in which the copolymer is a block copolymer or a grafted copolymer.

33. Use according to any one of the preceding claims, in which the polymer represents from 0.5% to 80% and better still from 5% to 40% of the total weight of the composition.

34. Use according to any one of the preceding claims, in which the gelling polymer/silicone oil mass ratio is from 0.1% to 50%.

35. Use according to any one of the preceding claims, in which the liquid fatty phase represents from 5% to 99% of the total weight of the composition and better still from 20% to 75%.

36. Use according to one of the preceding claims, **characterized in that** the composition contains at least one cosmetic or dermatological active agent.

37. Use according to any one of the preceding claims, **characterized in that** the composition contains at least one additive chosen from water, antioxidants, essential oils, preserving agents, fragrances, liposoluble polymers, especially hydrocarbon-based liposoluble polymers such as polyalkylenes or polyvinyl laurate, aqueous-phase gelling agents, liquid-fatty-phase gelling agents, waxes, gums, resins, surfactants, for instance trioleyl phosphate, additional cosmetic or dermatological active agents chosen from the group consisting of emollients, moisturizers, vitamins, liquid lanolin, essential fatty acids, lipophilic sunscreens or sunscreens that are soluble in polyols, and mixtures thereof.

38. Composition according to any one of the preceding claims, **characterized in that** the composition is in the form of a transparent anhydrous rigid gel, and especially in the form of a transparent anhydrous stick.

39. Use according to claim 1, in which the composition is a structured solid makeup composition for the skin, the lips and/or integuments, containing at least one pigment and/or a gloss in an amount that is sufficient to make up the skin, the lips and/or integuments, and a liquid continuous fatty phase, said composition comprising at least 10 wt.% of a non-volatile oil and/or a pasty or viscous product, said composition being in the form of a solid, and the pigment and/or gloss, the fatty liquid phase and the polymer form a physiologically acceptable medium.

40. Use according to claim 39, in which the composition is a structured lipstick composition containing at least one pigment and/or a gloss in a sufficient quantity for making up the lips.

41. Use according to claim 38, in which the polymer comprises at least one moiety corresponding to one of the following formulae: formula (I) of claim 19, formula (II) of claim 21, formulae (III) and (IV) of claim 22, formula (VII) of claim 26, formula (VIII) of claim 28, formula (I) of claim 29 and formula (XIII) of claim 30.

42. Use according to any one of claims 1 to 41, **characterized in that** it is in the form of a mascara cake, an eyeliner, a foundation, a lipstick, a blusher, a deodorant product, a makeup-removing product, a makeup product for the body, an eyeshadow, a face powder or a concealer product.

43. Use according to claim 1, in which the composition is in the form of a makeup stick for the skin, the lips and/or integuments, and in particular the lips, containing at least one pigment in an amount that is sufficient to make up the skin, the lips and/or integuments, the composition comprising at least 10 wt.% of non-volatile oil and/or a pasty or viscous product, the pigment and/or the gloss, the fatty phase and the polymer forming a physiologically acceptable medium.

44. Use according to claim 1, in which the composition has a hardness of from 20 to 2,000 gf, preferably from 20 to 900 gf and better still from 20 to 600 gf.

45. Cosmetic process for limiting the migration of a cosmetic composition containing a liquid fatty phase comprising at least one silicone oil, which consists in structuring the said fatty phase with a sufficient amount of at least one polymer (homopolymer or copolymer) with a weight-average molecular mass ranging from 500 to 500,000, containing at least one moiety comprising:
- at least one polyorganosiloxane group, consisting of from 1 to 1,000 organosiloxane units in the chain of the moiety or in the form of a graft, and
- at least two groups capable of establishing hydrogen interactions, chosen from ester, amide, sulphonamide, carbamate, thiocarbamate, urea, thiourea, oxamido, guanidino and biguanidino groups, and combinations thereof, on condition that at least one of the groups is other than an ester group,
the polymer being solid at room temperature and soluble in the liquid fatty phase at a temperature of from 25 to 250°C,
the liquid fatty phase consisting partially or totally of volatile oil(s) with a flash point greater than or equal to 40°C and greater than the softening point of the gelling polymer, on condition that the composition comprises at least 10% by mass of a non-volatile oil and/or a pasty or viscous product.

## Patentansprüche

1. Verwendung einer kontinuierlichen flüssigen Fettphase, die mindestens ein Siliconöl enthält, das im Wesentlichen durch eine ausreichende Menge mindestens eines Polymers (Homopolymers oder Copolymers) mit einem Gewichtsmittel des Molekulargewichts von 500 bis 500 000 strukturiert ist, das mindestens eine Einheit aufweist, die ihrerseits aufweist:
- mindestens eine Polyorganosiloxangruppe, die aus 1 bis 1000 Organosiloxan-Einheiten in der Kette der Einheit oder in Form von Pfropfgruppen besteht, und
- mindestens zwei Gruppen, die zur Bildung von Wasserstoffbrücken befähigt und ausgewählt sind unter Estergruppen, Amidgruppen, Sulfonamidgruppen, Carbamatgruppen, Thiocarbamatgruppen, Harnstoffgruppen, Thioharnstoffgruppen, Oxamidogruppen, Guanidinogruppen und Biguanidinogruppen sowie Kombinationen dieser Gruppen,
mit der Maßgabe, dass mindestens eine der Gruppen von einer Estergruppe verschieden ist,
wobei das Polymer bei Umgebungstemperatur fest ist und bei einer Temperatur von 25 bis 250 °C in der flüssigen Fettphase löslich ist,
wobei die flüssige Fettphase teilweise oder vollständig aus einem oder mehreren flüchtigen Ölen besteht, deren Klärpunkt gleich oder höher als 40 °C ist und über der Erweichungstemperatur des gelbildenden Polymers liegt,
in einer kosmetischen Zusammensetzung zum Schminken, die feste Partikel enthält, die unter Füllstoffen, Pigmenten und/oder Perlglanzmitteln und Gemischen dieser Stoffe ausgewählt sind, oder zur Herstellung einer Zusammensetzung zum Schminken, die feste Partikel enthält, die unter Füllstoffen, Pigmenten und/oder Perlglanzmitteln und Gemischen dieser Stoffe ausgewählt sind, Wobei die Zusammensetzung physiologisch akzeptabel ist,
als Mittel zur Begrenzung der Migration der Zusammensetzung
mit der Maßgabe, dass die Zusammensetzung mindestens 10 Masse- % eines nichtflüchtigen Öls und/oder eines pastosen oder viskosen Produkts enthält.

2. Verwendung nach Anspruch 1, wobei die Fettphase ein Gemisch von Ölen ist, das mindestens ein nichtflüchtiges Siliconöl und mindestens ein flüchtiges Öl enthält, dessen Klärpunkt gleich oder höher als 40 °C ist und über der Erweichungstemperatur des gelbildenden Polymers liegt.

3. Verwendung nach Anspruch 2, wobei die flüssige Fettphase mindestens ein nichtflüchtiges Öl enthält, das kein Siliconöl ist.

4. Verwendung nach Anspruch 1, wobei die Fettphase ausschließlich aus einem oder mehreren flüchtigen Siliconölen besteht, deren Klärpunkt gleich oder höher als 40 °C ist und über der Erweichungstemperatur des gelbildenden Polymers liegt.

5. Verwendung nach Anspruch 2 oder 3, wobei das flüchtige Öl ein Siliconöl ist.

6. Verwendung nach Anspruch 2 oder 3, wobei das flüchtige Öl kein Siliconöl ist.

7. Verwendung nach Anspruch 2 oder 3, wobei das Gemisch mindestens ein flüchtiges Siliconöl und mindestens ein flüchtiges Öl, das kein Siliconöl ist, enthält, deren Klärpunkte gleich oder höher als 40 °C sind und über der Erweichungstemperatur des gelbildenden Polymers liegen.

8. Verwendung nach einem der Ansprüche 4, 5 und 7, wobei das flüchtige Siliconöl ausgewählt ist aus der Gruppe, die aus folgenden Verbindungen besteht: Octyltrimethicon, Hexyltrimethicon, Octamethylcyclotetrasiloxan D4, Dodecamethycyclohexasiloxan D6, Heptamethyloctyltrisiloxan, Decamethyltetrasiloxan, Dodecamethylpentasiloxan, Polydimethylsiloxan von 1,5 cSt, Polydimethylsiloxan von 2 cSt, Polymethylsiloxan von 3 cSt und Polydimethylsiloxan von 5 cSt sowie Gemische dieser Verbindungen.

9. Verwendung nach einem der Ansprüche 6 und 7, wobei das flüchtige Öl, das kein Siliconöl ist, aus der Gruppe ausgewählt ist, die aus folgenden Verbindungen besteht:
Isododecan, Isohexadecan, C₈-C₁₆-Isoparaffinen, Isodecylneopentanoat, Propylenglycol-n-butylether, 3-Ethoxypropionsäureethylester, Propylenglycolmethyletheracetat und Isohexylneopentanoat sowie Gemischen dieser Verbindungen.

10. Verwendung nach einem der vorhergehenden Ansprüche, **dadurch gekenzeichnet, dass** das flüchtige Öl einen Klärpunkt über 60 °C, bevorzugt über 80 °C und noch bevorzugter über 93 °C besitzt.

11. Verwendung nach einem der vorhergehenden Ansprüche, **dadurch gekennzeichnet, dass** das flüchtige Öl einen Klärpunkt gleich oder niedriger als 160 °C und bevorzugt gleich oder niedriger als 135 °C besitzt.

12. Verwendung nach einem der vorhergehenden Ansprüche, wobei die flüssige Fettphase mindestens 40 Gew.-% und noch günstiger mindestens 50 Gew.- % Siliconöl enthält.

13. Verwendung nach einem der vorhergehenden Ansprüche, wobei das flüchtige Öl 3 bis 89,4 % des Gesamtgewichts der Zusammensetzung ausmacht.

14. Verwendung nach einem der vorhergehenden Ansprüche, die ferner ein Färbemittel umfaßt.

15. Verwendung nach Anspruch 1, wobei die festen Partikel hydrophobe Partikel sind.

16. Verwendung nach Anspruch 1, wobei die festen Partikel hydrophile Partikel sind, die mit einem Film einer hydrophoben Verbindung ummantelt sind.

17. Verwendung nach Anspruch 1, wobei die festen Partikel hydrophile Partikel sind und die Zusammensetzung ferner ein amphiphiles Silicon enthält.

18. Verwendung nach Anspruch 1, wobei die Partikel Pigmente sind, die unter Zinkoxiden, Eisenoxiden und Titanoxiden sowie Gemischen dieser Verbindungen ausgewählt sind.

19. Verwendung nach einem der Ansprüche 1 bis 18, wobei das als Gelbildungsmittel verwendete Polymer mindestens eine Einheit der Formel 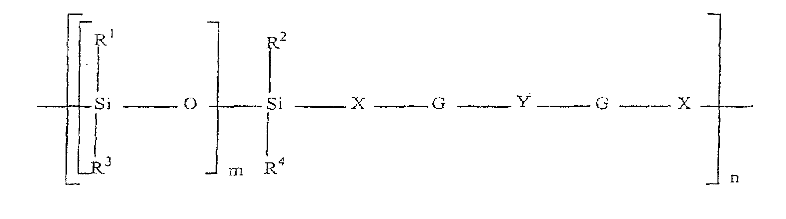 aufweist,
in der bedeuten:
1) R¹, R², R³ und R⁴, die gleich oder verschieden sind, eine Gruppe, die ausgewählt ist unter:
- geradkettigen, verzweigten oder cyclischen, gesättigten oder ungesättigten C₁-C₄₀-Kohlenwasserstoffgruppen, die in ihrer Kette ein oder mehrere Sauerstoffatome, Schwefelatome und/oder Stickstoffatome enthalten können und teilweise oder ganz mit Fluoratomen substituiert sein können,
- C₆-C₁₀-Arylgruppen, die gegebenenfalls mit einer oder mehreren C₁-C₄-Alkylgruppen substituiert sind,
- Polyorganosiloxanketten, die gegebenenfalls ein oder mehrere Sauerstoffatome, Schwefelatome und/oder Stickstoffatome enthalten;
2) X, die gleich oder verschieden sind, eine geradkettige oder verzweigte C₁-C₃₀-Alkylendiylgruppe, die in ihrer Kette ein oder mehrere Sauerstoffatome und/oder Stickstoffatome enthalten kann;
3) Y eine zweiwertige gesättigte oder ungesättigte C₁-C₅₀gruppe, die eine geradkettige oder verzweigte Alkylengruppe, eine Arylengruppe, eine Cycloalkylengruppe, eine Alkylarylengruppe oder eine Arylalkylengruppe ist und ein oder mehrere Sauerstoffatome, Schwefelatome und/oder Stickstoffatome aufweisen kann und/oder eines der folgenden Atome oder eine der folgenden Atomgruppen als Substituenten enthalten kann: Fluor, Hydroxy, C₃-C₈-Cycloalkyl, C₁-C₄₀-Alkyl, C₅-C₁₀-Aryl, Phenyl, das gegebenenfalls mit 1 bis 3 C₁-C₃-Alkylgruppen substituiert ist, C₁-C₃-Hydroxyalkyl und C₁-C₆-Aminoalkyl, oder
4) Y eine Gruppe der Formel 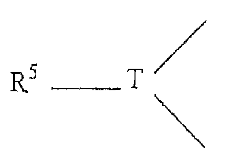 in der bedeuten:
- T eine geradkettige oder verzweigte, gesättigte oder ungesättigte dreiwertige oder vierwertige C₃-C₂₄-Kohlenwasserstoffgruppe, die gegebenenfalls mit einer Polyorganosiloxankette substituiert ist und ein oder mehrere Atome enthalten kann, die unter O, N und S ausgewählt sind, oder T ein dreiwertiges Atom, das unter N, P und Al ausgwählt ist, und
- R⁵ eine geradkettige oder verzweigte C₁-C₅₀-Alkylgruppe oder eine Polyorganosiloxankette, die eine oder mehrere Estergruppen, Amidgruppen, Urethangruppen, Thiocarbamatgruppen, Harnstoffgruppen, Thioharnstoffgruppen und/oder Sulfonamidgruppen aufweisen kann und die gegebenenfalls an eine andere Kette des Polymers gebunden sein kann,
5) G, die gleich oder verschieden sind, zweiwertige Gruppen, die ausgewählt sind unter 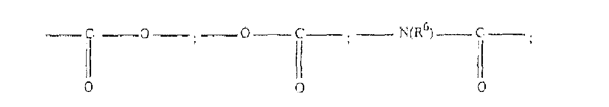 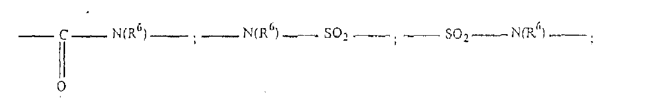 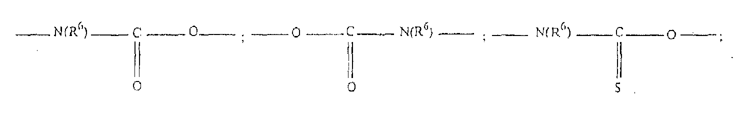 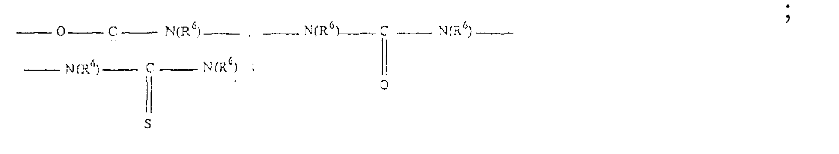 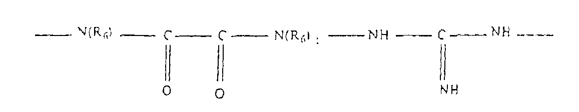 und 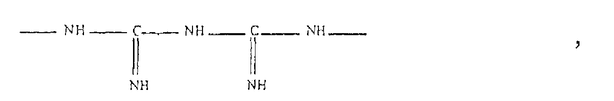 worin R⁶ ein Wasserstoffatom oder eine geradkettige oder verzweigte C₁-C₂₀-Alkylgruppe bedeutet, mit der Maßgabe, dass mindestens 50 % der Gruppen R⁶ des Polymers ein Wasserstoffatom darstellen und mindestens zwei der Gruppen G des Polymers eine andere Gruppe darstellen als 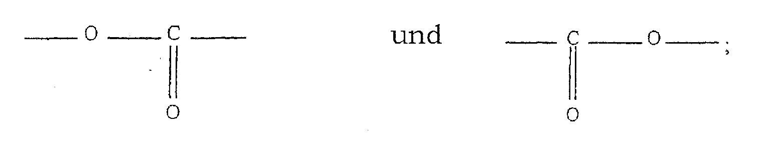
6) n eine ganze Zahl von 2 bis 500 und bevorzugt 2 bis 200 und
m eine ganze Zahl von 1 bis 1000, bevorzugt von 1 bis 700 und noch günstiger von 6 bis 200.

20. Verwendung nach Anspruch 19, wobei Y eine Gruppe bedeutet, die ausgewählt ist unter:
a) geradkettigen C₁-C₂₀-Alkylengruppen, vorzugsweise geradkettigen C₁-C₁₀-Alkylengruppen,
b) verzweigten C₃₀-C₅₆-Alkylengruppen, die Ringe sowie nichtkonjugierte ungesättigte Bindungen aufweisen können,
c) C₅₋C₆-Cycloalkylengruppen,
d) Phenylengruppen, die gegebenenfalls mit einer oder mehreren C₁-C₄₀-Alkylgruppen substituiert sind,
e) C₁-C₂₀-Alkylengruppen, die 1 bis 5 Amidgruppen enthalten,
f) C₁-C₂₀-Alkylengruppen, die einen oder mehrere Substituenten aufweisen, die ausgewählt sind unter Hydroxygruppen, C₃-C₈-Cycloalkangruppen, C₁-C₃-Hydroxyalkylgruppen und C₁-C₆-Alkylamingruppen, und
g) Polyorganosiloxangruppen der Formel 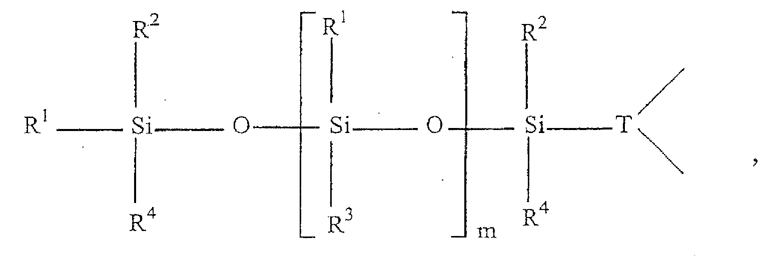
worin R¹, R², R³, R⁴, T und m wie oben definiert sind.

21. Verwendung nach einem der Ansprüche 1 bis 18, wobei das als Gelbildungsmittel verwendete Polymer mindestens eine Einheit der Formel (II) aufweist: 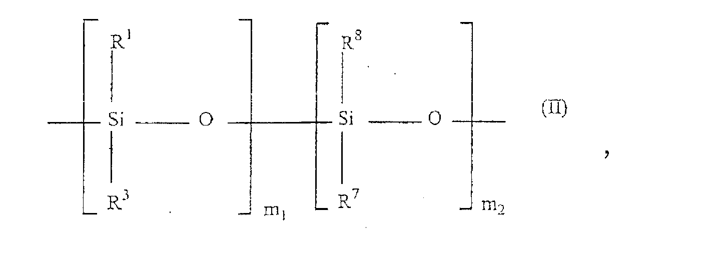 worin bedeuten:
- R¹ und R², die gleich oder verschieden sind, dasselbe wie oben für Formel (I) in Anspruch 19 definiert,
- R⁷ eine Gruppe wie oben für R¹ und R³ definiert, oder die Gruppe der Formel -X-G-R⁹, in der X und G wie oben für Formel (I) in Anspruch 19 definiert sind und R⁹ ein Wasserstoffatom oder eine geradkettige, verzweigte oder cyclische, gesättigte oder ungesättigte C₁-C₅₀-Kohlenwasserstoffgruppe bedeutet, die gegebenenfalls in ihrer Kette ein oder mehrere Atome aufweist, die unter O, S und N ausgewählt sind und die gegebenenfalls mit einem oder mehreren Fluoratomen und/oder einer oder mehreren Hydroxygruppen substituiert ist, oder eine Phenylgruppe darstellt, die gegebenenfalls mit einer oder mehreren C₁C₄-Alkylgruppen substituiert ist,
- R⁸ die Gruppe der Formel -X-G-R⁹, in der S, G und R⁹ wie oben definiert sind,
- m₁ eine ganze Zahl von 1 bis 998 und
- m₂ eine ganze Zahl von 2 bis 500.

22. Verwendung nach Anspruch 19, wobei das Polymer mindestens eine Einheit der Formel (III) oder (IV) aufweist: 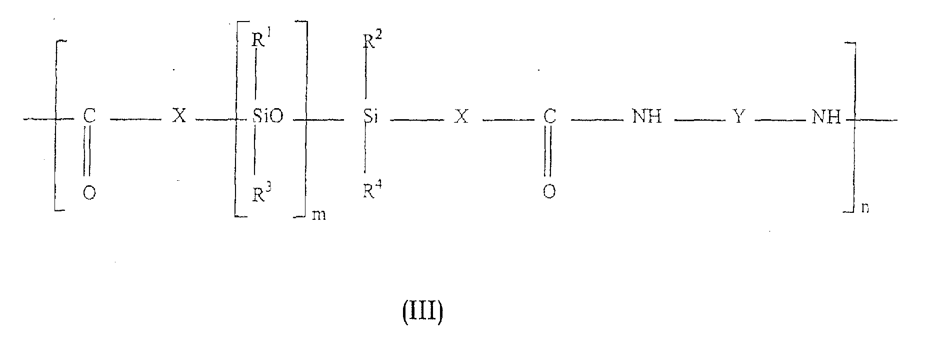 oder 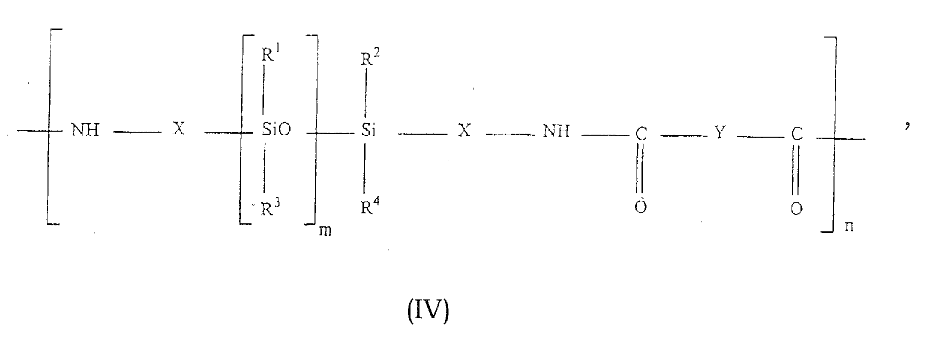 worin R¹, R², R³, R⁴, X, Y, m und n wie in Anspruch 19 definiert sind.

23. Verwendung nach einem der Ansprüche 19 und 20, wobei X und/oder Y eine Alkylengruppe darstellen, die in ihrem Alkylenteil mindestens eines der nachstehenden Elemente enthält:
1.) 1 bis 5 Amidgruppen, Harnstoffgruppen oder Carbamatgruppen,
2.) eine C₅- oder C₆-Cycloalkylgruppe und
3.) eine Phenylengruppe, die gegebenenfalls mit 1 bis 3 gleichen oder verschiedenen C₁-C₃-Alkylgruppen und/oder mindestens einem Element substituiert ist, was aus der Gruppe ausgewählt ist, die besteht aus:
- einer Hydroxygruppe,
- einer C₃-C₈-Cycloalkylgruppe,
- einer bis drei C₁-C₄₀-Alkylgruppen,
- einer Phenylgruppe, die gegebenenfalls mit einer bis drei C₁-C₃-Alkylgruppen substituiert ist,
- einer C₁-C₃-Hydroxyalkylgruppe und
- einer C₁-C₆-Aminoalkylgruppe.

24. Verwendung nach einem der Ansprüche 19 bis 22, wobei Y die Gruppierung 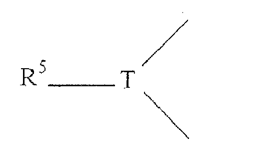 darstellt,
worin bedeuten:
R⁵ eine Polyorganosiloxankette
und
T eine Gruppe der Formel 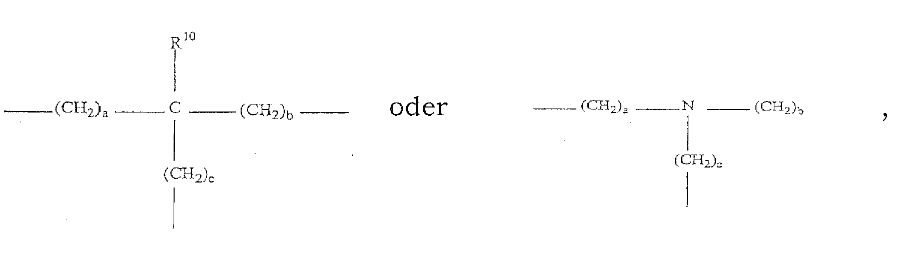
worin a, b und c unabhängig ganze Zahlen von 1 bis 10 bedeuten und R¹⁰ ein Wasserstoffatom oder eine Gruppe ist, wie sie für R¹, R², R³ und R⁴ in Anspruch 19 definiert ist.

25. Verwendung nach einem der Ansprüche 19 bis 22, wobei R¹, R², R³ und R⁴ unabhängig eine geradkettige oder verzweigte C₁-C₄₀-Alkylgruppe, bevorzugt CH₃, C₂H₅, n-C₃H₇ oder Isopropyl, eine Polyorganosiloxankette oder eine Phenylgruppe, die gegebenenfalls mit 1 bis 3 Methylgruppen oder Ethylgruppen substituiert ist, bedeuten.

26. Verwendung nach Anspruch 1, wobei das als Gelbildungsmittel verwendete Polymer mindestens eine Einheit der Formel 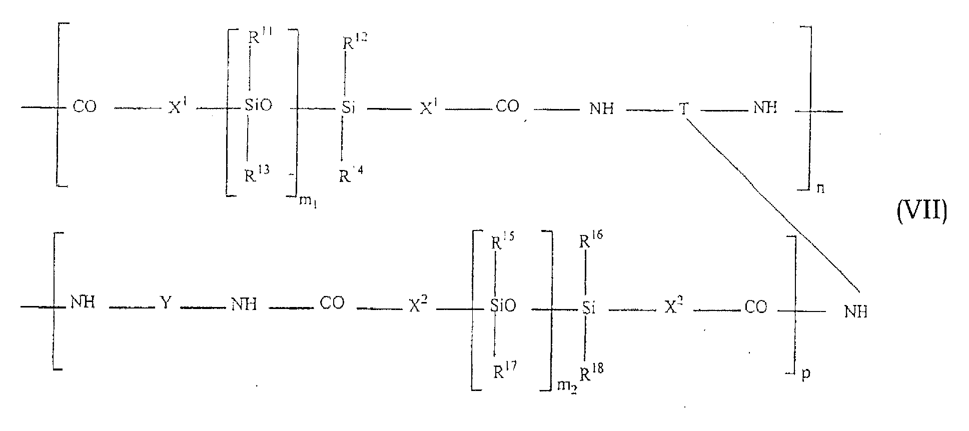 enthält,
in der bedeuten:
X¹ und X², die gleich oder verschieden sind, dasselbe wie X in Anspruch 19,
n, Y und T dasselbe wie in Anspruch 19 definiert,
R¹¹ bis R¹⁸ Gruppen, die unter den gleichen Gruppen wie R¹
bis R⁴ von Anspruch 19 ausgewählt sind,
m₁ und m₂ Zahlen im Bereich von 1 bis 1000
und
p eine ganze Zahl von 2 bis 500.

27. Verwendung nach Anspruch 26, wobei bedeuten:
- p eine Zahl im Bereich von 1 bis 25 und noch günstiger im Bereich von 1 bis 7,
- R¹¹ bis R¹⁸ Methylgruppen,
- T eine der nachstehenden Formeln: 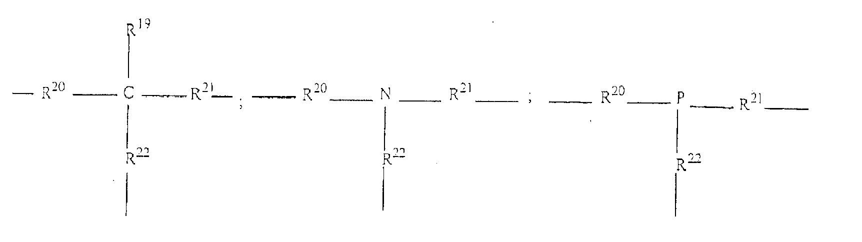 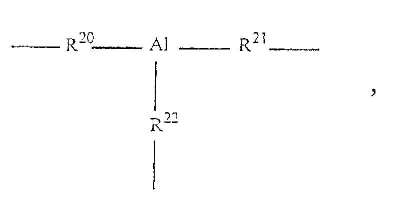
worin bedeuten:
R¹⁹ ein Wasserstoffatom oder eine Gruppe, die unter den für R¹ bis R⁴ definierten Gruppen ausgewählt ist,
und
R²⁰, R²¹ und R²² unabhängig geradkettige oder verzweigte Alkylengruppen und noch bevorzugter Gruppen der Formel 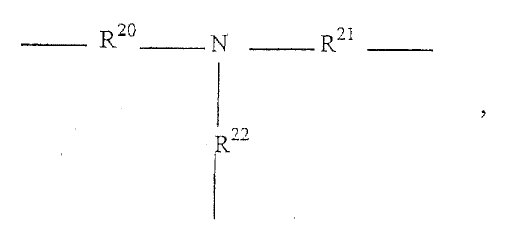
wobei R²⁰, R²¹ und R²² insbesondere -CH₂-CH₂- bedeuten,
- m₁ und m₂ eine Zahl im Bereich von 15 bis 500 und noch günstiger im Bereich von 15 bis 45,
- X¹ und X² -(CH₂)₁₀
und
- Y -CH₂-.

28. Verwendung nach einem der Ansprüche 1 bis 18, wobei das Polymer mindestens eine Einheit der nachstehenden Formel 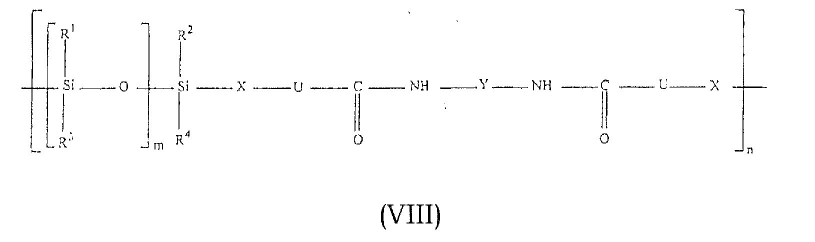 aufweist,
worin bedeuten:
R¹, R², R³, R⁴, X, Y, m und n das gleiche wie für Formel (I) in Anspruch 19 angegeben
und
U -O- oder -NH-
oder
Y eine cycloaliphatische oder aromatische C₅-C₁₂gruppe, die mit einer C₁-C₁₅-Alkylgruppe oder einer C₅-C₁₀-Arylgruppe substituiert sein kann, z.B. eine Gruppe, die ausgewählt ist unter
Methylen-4, 4-biscyclohexyl, der von Isophorondiisocyanat abgeleiteten Gruppe, 2,4-Tolylen und 2,6-Tolylen, 1,5-Naphthylen, p-Phenylen und
4,4'-Biphenylenmethan,
oder
Y eine geradkettige oder verzweigte C₁-C₄₀-Alkylengruppe oder eine C₄-C₁₂-Cycloalkylengruppe
oder
Y eine Polyurethansequenz oder Polyharnstoffsequenz, die der Kondensation von mehreren Diisocyanatmolekülen mit einem oder mehreren Kupplermolekülen vom Dioltyp oder vom Diamintyp der Formel 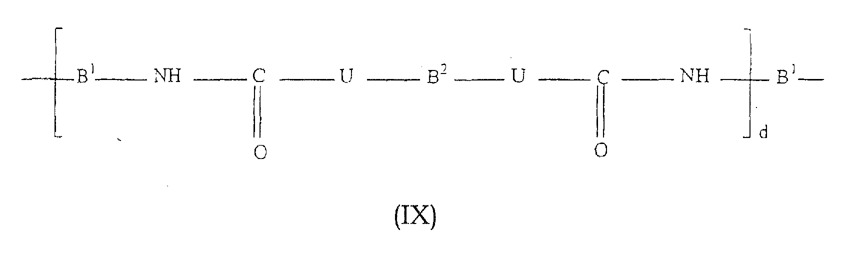 entspricht,
worin
B¹ eine unter den oben für Y angegebenen Gruppen ausgewählte Gruppe ist,
U -O- oder -NH- bedeutet
und
B² ausgewählt ist unter:
• geradkettigen oder verzweigten C₁-C₄₀-Alkylengruppen, die gegebenenfalls eine ionisierbare Gruppe, wie etwa eine Carbonsäuregruppe oder Sulfonsäuregruppe, oder eine neutralsierbare oder quaternisierbare tertiäre Amingruppe tragen können,
• C₅-C₁₂-Cycloalkylengruppen, die gegebenenfalls Alkylsubstituenten tragen, z.B. eine bis drei Methylgruppen oder Ethylgruppen, oder Alkylengruppen, z.B. die Gruppe des Diols Cyclohexandimethanol,
• Phenylengruppen, die gegebenenfalls C₁-C₃-Alkylsubstituenten tragen können,
und
• Gruppen der Formel 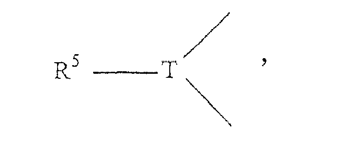
worin bedeuten:
T eine dreiwertige Kohlenwasserstoffgruppe, die ein oder mehrere Heteroatome, wie Sauerstoff, Schwefel- und Stickstoff, aufweisen kann,
und
R⁵ eine Polyorganosiloxankette oder eine geradkettige oder verzweigte C₁-C₅₀-Alkylkette.

29. Verwendung nach einem der Ansprüche 1 bis 18, wobei das Polymer mindestens eine Einheit der Formel 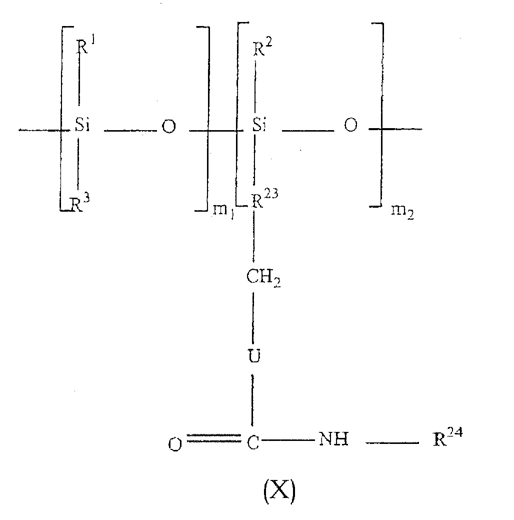 aufweist,
in der bedeuten:
- R¹, R², m₁ und m₂ das gleiche wie oben für Formel (I) angegeben,
- U O oder NH,
- R²³ eine C₁-C₄₀-Alkylengruppe, die gegebenenfalls ein oder mehrere unter O und N ausgewählte Heteroatome aufweist, oder eine Phenylengruppe
und
- R²⁴ eine Gruppe, die ausgewählt ist unter geradkettigen, verzweigten oder cyclischen, gesättigten oder ungesättigten C₁-C₅₀-Alkylgruppen und Phenylgruppen, die gegebenenfalls mit einer bis drei C₁-C₃-Alkylgruppen substituiert sind.

30. Verwendung nach einem der Ansprüche 1 bis 18, wobei das als Gelbildungsmittel verwendete Polymer mindestens eine Einheit der Formel 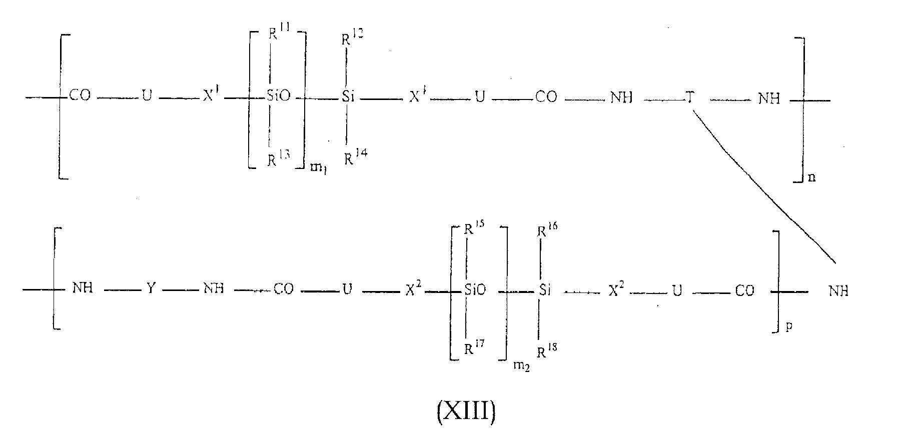 aufweist,
in der bedeuten:
X¹ und X², die gleich oder verschieden sind, dasselbe, was für X in Anspruch 19 angegeben ist,
n, Y und T dasselbe wie in Anspruch 19 definiert,
R¹¹ bis R¹⁸ Gruppen, die ausgewählt sind unter den gleichen Gruppen, wie sie für R¹ bis R⁴ in Anspruch 19 angegeben sind,
m₁ und m₂ Zahlen im Bereich von 1 bis 1000
und
p eine ganze Zahl von 2 bis 500.

31. Verwendung nach einem der Ansprüche 19 bis 30, wobei das als Gelbildungsmittel verwendete Polymer ferner eine Kohlenwasserstoffeinheit aufweist, die zwei Gruppen aufweist, die zur Bildung von Wasserstoffbrücken befähigt und ausgewählt sind unter Estergruppen, Amidgruppen, Sulfonamidgruppen, Carbamatgruppen, Thiocarbamatgruppen, Harnstoffgruppen, Thioharnstoffgruppen sowie Kombinationen dieser Gruppen.

32. Verwendung nach Anspruch 30, wobei das Copolymer ein Blockcopolymer, ein sequentielles Copolymer oder ein Pfropfcopolymer ist.

33. Verwendung nach einem der vorhergehenden Ansprüche, wobei das Polymer 0,5 bis 80 % und noch günstiger 5 bis 40 % des Gesamtgewichts der Zusammensetzung ausmacht.

34. Verwendung nach einem der vorhergehenden Ansprüche, wobei das Massenverhältnis von gelbildendem Polymer zu einem oder mehreren Siliconölen 0,1 bis 50 % beträgt.

35. Verwendung nach einem der vorhergehenden Ansprüche, wobei die flüssige Fettphase 5 bis 99 % und noch günstiger 20 bis 75 % des Gesamtgewichts der Zusammensetzung ausmacht.

36. Verwendung nach einem der vorhergehenden Ansprüche, **dadurch gekennzeichnet, dass** die Zusammensetzung ferner mindestens einen kosmetischen oder dermatologischen Wirkstoff enthält.

37. Verwendung nach einem der vorhergehenden Ansprüche, **dadurch gekennzeichnet, dass** die Zusammensetzung mindestens ein Additiv enthält, das ausgewählt ist unter Wasser, Antioxidantien, essentiellen Ölen, Konservierungsmitteln, Parfums, fettlöslichen Polymeren, insbesondere Kohlenwasserstoffpolymeren, wie Polyalkylenen oder Vinylpolylaurat, Gelbildnern für die wässerige Phase, Gelbildnern für die flüssige Fettphase, Wachsen, Gummen, Harzen, grenzflächenaktiven Mitteln, wie Trioleylphosphat, zusätzlichen kosmetischen oder dermatologischen Wirkstoffen, die ausgewählt sind aus der Gruppe, die besteht aus Emollientien, Hydratisierungsmitteln, Vitaminen, flüssigem Lanolin, essentiellen Fettsäuren und lipophilen oder in Polyolen löslichen Sonnenfiltern sowie Gemischen dieser Verbindungen.

38. Verwendung nach einem der vorhergehenden Ansprüche, **dadurch gekennzeichnet, dass** die Zusammensetzung in Form eines transparenten, wasserfreien, festen Gels und insbesondere eines transparenten wasserfreien Stifts vorliegt.

39. Verwendung nach Anspruch 1, wobei die Zusammensetzung eine feste, strukturierte Zusammensetzung zum Schminken der Haut, der Lippen und/oder der Hautanhangsgebilde ist und mindestens ein Pigment und/oder ein Perlglanzmittel in einer zum Schminken der Haut, der Lippen und/oder der Hautanhangsgebilde ausreichenden Menge sowie eine kontinuierliche flüssige Fettphase enthält, wobei die Zusammensetzung mindestens 10 Masse- % eines nichtflüchtigen Öls und/oder eines pastosen oder viskosen Produkts enthält und die Zusammensetzung in Form eines Feststoffs vorliegt und das Pigment und/oder das Perlglanzmittel, die flüssige Fettphase und das Polymer ein physiologisch akzeptables Milieu bilden.

40. Verwendung nach Anspruch 39, wobei die Zusammensetzung eine strukturierte Zusammensetzung für Lippenstifte ist, die mindestens ein Pigment und/oder ein Perlglanzmittel in einer zum Schminken der Lippen ausreichenden Menge enthält.

41. Verwendung nach Anspruch 38, wobei das Polymer mindestens eine Einheit einer der nachstehenden Formeln enthält: Formel (I) von Anspruch 19, Formel (II) von Anspruch 21, Formeln (III) und (IV) von Anspruch 22, Formel (VII) von Anspruch 26, Formel (VIII) von Anspruch 28, Formel (X) von Anspruch 29 und Formel (XIII) von Anspruch 30.

42. Verwendung nach einem der Ansprüche 1 bis 41, **dadurch gekennzeichnet, dass** die Zusammensetzung als Mascara-Block, als Eyeliner, als Make-up, als Lippenstift, als Blush, als Produkt zum Schminken des Körpers, als Lidschatten oder Rouge oder als Produkt gegen Augenringe vorliegt.

43. Verwendung nach Anspruch 1, wobei die Zusammensetzung in Form eines Stifts zum Schminken der Haut, der Lippen und/oder der Hautanhangsgebilde und insbesondere der Lippen vorliegt und mindestens ein Pigment und/oder ein Perlglanzmittel in einer zum Schminken der Haut, der Lippen und/oder der Hautanhangsgebilde ausreichenden Menge enthält, wobei die Zusammensetzung mindestens 10 Masse- % eines nichtflüchtigen Öls und/oder eines pastosen oder viskosen Produkts enthält und wobei das Pigment und/oder das Perlglanzmittel, die Fettphase und das Polymer ein physiologisch akzeptables Milieu bilden.

44. Verwendung nach Anspruch 1, wobei die Zusammensetzung eine Härte von 20 bis 2000 gf, bevorzugt von 20 bis 900 gf und noch günstiger von 20 bis 600 gf aufweist.

45. Kosmetisches Verfahren zur Begrenzung der Migration einer kosmetischen Zusammensetzung zum Schminken, die feste Partikel enthält, die unter Füllstoffen, Pigmenten und/oder Perlglanzmitteln und Gemischen dieser Stoffe ausgewählt sind,
wobei die Zusammensetzung eine flüssige Fettphase enthält, die mindestens ein Siliconöl enthält,
das darin besteht, die Fettphase durch eine ausreichende Menge mindestens eines Polymers (Homopolymers oder Copolymers) mit einem Gewichtsmittel des Molekulargewichts von 500 bis 500 000 zu strukturieren, das mindestens eine Einheit aufweist, die ihrerseits aufweist:
- mindestens eine Polyorganosiloxangruppe, die aus 1 bis 1000 Organosiloxan-Einheiten in der Kette der Einheit oder in Form von Pfropfgruppen besteht, und
- mindestens zwei Gruppen, die zur Bildung von Wasserstoffbrücken befähigt und ausgewählt sind unter Estergruppen, Amidgruppen, Sulfonamidgruppen, Carbamatgruppen, Thiocarbamatgruppen, Harnstoffgruppen, Thioharnstoffgruppen, Oxamidogruppen, Guanidinogruppen und Biguanidinogruppen sowie Kombinationen dieser Gruppen,
mit der Maßgabe, dass mindestens eine der Gruppen von einer Estergruppe verschieden ist,
wobei das Polymer bei Umgebungstemperatur fest ist und bei einer Temperatur von 25 bis 250 °C in der flüssigen Fettphase löslich ist,
wobei die flüssige Fettphase teilweise oder vollständig aus einem oder mehreren flüchtigen Ölen besteht, deren Klärpunkt gleich oder höher als 40 °C ist und über der Erweichungstemperatur des gelbildenden Polymers liegt, mit der Maßgabe, dass die Zusammensetzung mindestens 10 Masse- % eines nichtflüchtigen Öls und/oder eines pastosen oder viskosen Produkts enthält.
